# EUROPEAN PATENT SPECIFICATION

(11) **EP 2 887 974 B1**
(45) Date of publication and mention of the grant of the patent: **26.07.2017**
(21) Application number: 13756075.1
(22) Date of filing: 23.08.2013
(51) Int. Cl.: A61L 29/08, A61L 29/16

(54) **BALLOON SURFACE COATING FOR VALVULOPLASTY**
BALLONOBERFLÄCHENBESCHICHTUNG FÜR VALVULOPLASTIE
REVÊTEMENT DE SURFACE POUR UN BALLONNET POUR VALVULOPLASTIE

(30) Priority: 23.08.2012 WO PCT/EP2012/066458
(43) Date of publication of application: 01.07.2015
(73) Proprietor: Cardionovum GmbH, 53227 Bonn (DE)
(72) Inventor: ORLOWSKI, Michael, 53173 Bonn (DE)
(74) Representative: Arth, Hans-Lothar
(86) International application number: PCT/EP2013/067592
(87) International publication number: WO 2014/029889

(56) References cited:
- WO-A2-2008/139232
- US-A1- 2008 255 510

## Description

The present invention is directed to valvuloplasty catheter balloons and balloon catheters for valvuloplasty having such a catheter balloon coated with at least one layer containing at least one antiproliferative, immunosuppressive, anti-angiogenic, anti-inflammatory, anti-restenotic and/or anti-thrombotic agent and a top coat consisting of a polyvinyl alcohol - polyethylene glycol graft copolymer as well as the use of these balloon catheters for the prevention of restenosis after valvuloplasty.

The heart is a myogenic muscular organ found in all animals with a circulatory system including vertebrates. It is the most heavily worked muscle in the body contracting about three billion times during the course of a human lifetime as the heart is responsible for pumping blood throughout the blood vessels by repeated, rhythmic contractions. It consists of four chambers, two upper atria and two lower ventricles. The heart has four valves. The two atrioventricular (AV) valves, which are between the atria and the ventricles, are the mitral valve and the tricuspid valve. The two semilunar (SL) valves, which are in the arteries leaving the heart, are the aortic valve and the pulmonary valve. The valves have tissue flaps that open and close with each heartbeat and maintain coordinated unidirectional blood flow from the upper atria to the lower ventricles. The valves are attached to the subvalvular apparatus which comprises the papillary muscles and the chordae tendineae. The function of the subvalvular apparatus is to keep the valves from prolapsing into the atria when they close. The opening and closure of the valves is caused entirely by the pressure gradient across the valve. A form of heart disease occurs when a valve malfunctions and allows some blood to flow in the wrong direction. This is called regurgitation.

Valves can be affected by several processes. Mitral stenosis is a valvular heart disease characterized by the narrowing of the orifice of the mitral valve of the heart. In aortic insufficiency, also called aortic regurgitation, the aortic valve is incompetent and blood flows passively back to the heart in the wrong direction. In contrast, the valve fails to open fully in aortic stenosis, thereby obstructing blood flow out from the heart. Aortic valve stenosis is usually defined by restricted systolic opening of the valve leaflets, with a mean transvalvular pressure gradient of at least 10 mm Hg. Symptoms of aortic stenosis include progressive shortness of breath on exertion (exertional dyspnoea). This dyspnoea may be so subtle that affected persons are not consciously aware of but may inadvertently cut down on exertional activities. More severe symptoms include syncope, chest pain and angina pectoris that could lead to heart failure, endocarditis and/or sudden cardiac death.

Aortic valve stenosis is the most significant valvular disease in the Western world. Its incidence is expected to grow with the continuing increase in longevity. Severe aortic valve stenosis is associated with severe morbidity. Affected persons' death may occur within two to three years of symptom onset. Aortic valve replacement is the definite therapy for severe aortic stenosis. A balloon valvuloplasty of the stenotic aortic valve is always performed before the prosthetic valve is implanted. A balloon catheter is placed either transarterially or transapically by way of the delivery catheter and then expanded under tachycardic ventricular.

Mitral valvuloplasty is a minimally invasive therapeutic procedure to treat mitral stenosis by dilating the valve using a balloon catheter. A catheter with a special balloon is used. The balloon is sub-divided into three segments and is dilated in three stages. First the distal portion in the left ventricle is inflated. Second the proximal portion is dilated, in order to fix the centre segment at the valve orifice. Finally, the central section is inflated, this should take no longer than 15 seconds since full inflation obstructs the valve and causes congestion, leading to circulatory arrest.

Balloon valvuloplasty as the sole treatment plays a very limited role in adults because its efficacy is low and complication rate is high. Restenosis and clinical deterioration occur within 6 to 12 months in most patients. Nevertheless without these complications it would be favourable because of its minimal invasive character. The problem of restenosis in aortic valve stenosis was recently addressed by the European patent application EP08750817.2 describing a valvuloplasty catheter balloon coated with an anti-restenotic agent. Nevertheless the time period a valvuloplasty balloon is dilated should be as short as possible. The time period available is even shorter than during angioplasty. Therefore there is a need for compositions of active agent eluting systems which release the active agent in very short times. The time of the balloon inflation for angioplasty is around 30 seconds. The time of balloon inflation in balloon valvuloplasty cannot exceed 10 - 15 seconds. Such an express release of active agents during valvuloplasty is one objective of the present invention.

Furthermore, balloon valvuloplasty can be considered as a bridge to surgery in haemodynamically unstable patients who are at high risk for surgery. Also, patients with symptomatic severe aortic stenosis who require urgent major non-cardiac surgery may be eligible for balloon valvuloplasty. Occasionally balloon valvuloplasty could be considered as a palliative measure in individual cases when surgery is contraindicated because of severe co-morbidity. Moreover, balloon angioplasty plays an important role in the paediatric population.

Nowadays, it is known that active agents can be applied to an angioplasty balloon catheter with various matrix-substances, including substances such as the terpenoid shellolic acid. The active agents are released during the balloon inflation at the vessel stenosis, in order to penetrate the arterial wall segment, in order to evolve their antiproliferative and anti-inflammatory effects on the smooth muscle cells and to suppress proliferation in the vessel lumen.

The international patent application WO 2004/028582 A1 discloses multifold balloons which are coated, especially within the folds, with a composition of a pharmacological agent and a contrast medium. A method for spray coating catheter balloons is described in WO 2004/006976 A1. WO 2008/255510 A1 discloses PTCA balloons with a coating comprising a therapeutic agent and a contrast agent for local delivery.

However, the present application provides a balloon catheter, wherein the entire balloon surface or parts thereof are coated, for balloon valvuloplasty. The active agent is only released during valvuloplasty which means it is a very short term application, preferably only 10 seconds. Surprisingly it has been found that a short term application is suitable to reduce restenosis also at a heart valve. The fact that short term application is suitable to reduce restenosis of a vessel is not transferrable to a valve as a valve has another structure and is another type of tissue. Further the pathology and composition of the plaque having mostly at lot of calcification may be different.

It is an objective of the present invention to apply at least one active agent onto an valvuloplasty catheter balloon in such a manner that a coating is created which is easily detachable from the balloon surface upon inflation of the catheter balloon and will be effectively transferred to the tissue of the heart valves so that a therapeutic effect concerning reduction of restenosis can be achieved best.

Said objective is solved by the technical teaching of the independent claims. Further advantageous embodiments of the invention result from the dependent claims, the description, the figures and the examples.

Surprisingly it has been found that a valvuloplasty catheter balloon comprising a coating with an active agent and a top coat of a polyvinyl alcohol - polyethylene glycol graft copolymer is suited for resolving said objective. Preferably the top coat does not comprise any active agent.

Thus the present invention relates to valvuloplasty catheter balloons with a coating comprising at least one active agent and a top coat consisting of a polyvinyl alcohol - polyethylene glycol graft copolymer or in other words the present invention relates to valvuloplasty catheter balloons with a coating of at least one active agent and a top coat consisting of a polyvinyl alcohol-polyethylene glycol graft copolymer. Still in other words the present invention is directed to valvuloplasty catheter balloons coated with at least one active agent and a top coat consisting of a polyvinyl alcohol-polyethylene glycol graft copolymer or alternatively to valvuloplasty catheter balloons coated with at least one active agent and a top coat on said active agent coat consisting of a polyvinyl alcohol-polyethylene glycol graft copolymer. This means the valvuloplasty catheter balloon with a surface coating having at least two layers: an active agent layer comprising at least one active agent and a top coat of a polyvinyl alcohol - polyethylene glycol graft copolymer.

The top coat consists preferably of a polyvinyl alcohol - polyethylene glycol graft copolymer and thus does preferably not contain any active substance or any other ingredient or component of the coating. The active agent layer or also named as active agent coat may consist of the pure active agent, preferably paclitaxel, or may comprise further components such as shellac, polyethylene glycol, D-α-tocopherol polyethylene glycol succinate or a polyethoxylated surfactant or a polyethoxylated emulsifier or shellac and a polyethoxylated surfactant or shellac and a polyethoxylated emulsifier or shellac and polyethylene glycol or shellac and D-α-tocopherol polyethylene glycol succinate or polyethylene glycol. The layer consisting of the active agent or comprising the active agent is below the top coat and can be directly on the balloon surface or can be applied on an additional base layer which is below the active agent layer and preferably the base layer is directly coated on the balloon surface.

Further it has been found that a valvuloplasty catheter balloon comprising a coating with an active agent, a top coat and optionally further comprising a polyethoxylated surfactant or a polyethoxylated emulsifier or D-α-tocopherol polyethylene glycol succinate is especially suited for resolving the above-mentioned objective.

The invention is also directed to a valvuloplasty catheter balloon with a coating comprising at least one active agent and optionally shellac or optionally a polyethoxylated surfactant or optionally D-α-tocopherol polyethylene glycol succinate and a top coat consisting of a polyvinyl alcohol - polyethylene glycol graft copolymer. It is preferred that the coating or layer with the active agent comprises further polyethylene glycol, D-α-tocopherol polyethylene glycol succinate, a polyethoxylated surfactant or a polyethoxylated emulsifier. The top coat is applied to protect the coating of the active agent from premature dissolution and mechanical damage. Therefore the top coat is advantageous, because it protects the coating from a "wash off' effect and saves it for the instant release of active agent at the position of action. But at the same time the top coat of a polyvinyl alcohol - polyethylene glycol graft copolymer allows release of the active agent during valvuloplasty.

Particularly preferred are valvuloplasty catheter balloons with a top coat of a polyvinyl alcohol - polyethylene glycol graft copolymer consisting of 75% polyvinyl alcohol units and 25% polyethylene glycol units. A polyethylene glycol chain forms a base onto which side chains of polyvinyl alcohol are grafted. Thereby the graft polymer comprises ethylene glycol monomer units and alcohol bound monomer units in a ratio of 25:75.
It is further preferred that the polyvinyl alcohol - polyethylene glycol graft copolymer has an average molecular weight within the range of 40,000 Daltons to 50,000 Daltons, a melting point of 190°C - 210°C, more preferably of 195°C - 205°C and most preferably a melting point of approximately 200°C and is represented by the following formula which shows a characteristic part of such a graft copolymer:

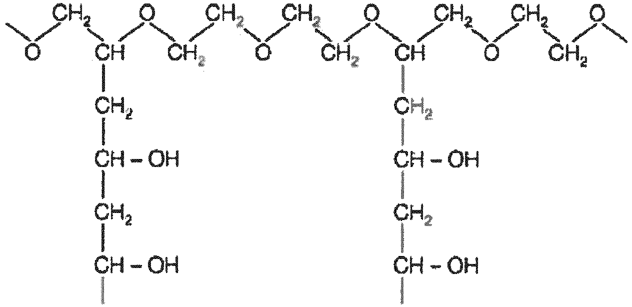

Said graft copolymer of the top coat may also comprise 0.1% to 0.5%, preferably 0.3%, colloidal silica to improve its flow properties. The preferred polyvinyl alcohol - polyethylene glycol graft copolymer dissolves readily in acidic, neutral and alkaline aqueous media, wherein the resulting solutions have a comparatively low viscosity.

The molecular weight of the graft copolymer is between 30,000 Daltons to 60,000 Daltons, more preferred between 40,000 Daltons to 50,000 Daltons, still more preferred between 42,000 and 48,000 Dalton and is most preferably around 44,000 - 46,000 Daltons. A clear colourless flexible film remains after evaporation of the water, when an aqueous solution of the graft copolymer is applied on a smooth surface. Other preferred variations of top coat components can comprise polyvinyl acetate dispersion (27%) stabilized with povidone (Polyvinylpyrrolidon, 2.7%) and sodium lauryl sulphate (0.3%) or methyl methacrylate and diethylaminoethyl methacrylate copolymer dispersion, wherein the solids concentration is approximately 30%.

A particularly preferred embodiment of the present invention is a valvuloplasty catheter balloon with a coating comprising paclitaxel and a top coat consisting of a polyvinyl alcohol - polyethylene glycol graft copolymer. In one embodiment of the invention the material of the top coat is also present in the active-agent layer where it is mixed with the active agent.

It is also possible to apply one or more additional additives as a carrier, excipient or second matrix substance to the surface of the valvuloplasty catheter balloon according to the invention. There are, for example, biologically compatible organic substances that improve the coating properties and increase the uptake of the active agent and especially of paclitaxel into the vessel, such as sugar and proteins like albumin or resins especially dammar, mastic, rosin or shellac. It is particularly preferred that the at least one additive is shellac. Nevertheless it is preferred that the coating of the valvuloplasty catheter balloon according to the invention does not comprise contrast agents, at least the active agent containing layer preferably does not contain contrast agents. Moreover the coating of the valvuloplasty catheter balloon of the present invention does not contain plasticizers such as acetyl tributyl citrate or acetyl triethyl citrate and does also not contain any citrate esters. Also excluded from the present invention are the additives sorbitol, sorbic acid, sorbates, sorbic acid esters, and any polysorbates. These substances are not used within the present invention.

Therefore the present invention relates to a valvuloplasty catheter balloon coated with a coating of at least one active agent and a top coat consisting of a polyvinyl alcohol - polyethylene glycol graft copolymer wherein the coating further comprises shellac. Particularly preferred as a matrix for the active agent, especially paclitaxel, is a combination of at least one polyethoxylated surfactant or a polyethoxylated emulsifier or D-α-tocopherol polyethylene glycol succinate together with shellac so that the present invention relates to a valvuloplasty catheter balloon coated with a coating of at least one active agent in a layer of D-α-tocopherol polyethylene glycol succinate together with shellac or at least one polyethoxylated surfactant together with shellac or a polyethoxylated emulsifier together with shellac and a top coat consisting of a polyvinyl alcohol-polyethylene glycol graft copolymer. Further preferred as a matrix for the active agent and especially paclitaxel is a combination of polyethylene glycol together with shellac so that the present invention relates to a valvuloplasty catheter balloon coated with a coating of at least one active agent in a layer of polyethylene glycol with shellac and a top coat consisting of a polyvinyl alcohol-polyethylene glycol graft copolymer. Also preferred as a matrix for the active agent and especially paclitaxel is a combination of at least one polyethoxylated surfactant or a polyethoxylated emulsifier together with polyethylene glycol so that the present invention relates to a valvuloplasty catheter balloon coated with a coating of at least one active agent in a layer of at least one polyethoxylated surfactant or a polyethoxylated emulsifier with polyethylene glycol and a top coat consisting of a polyvinyl alcohol-polyethylene glycol graft copolymer. Also preferred as a matrix for the active agent and especially paclitaxel is a combination of D-α-tocopherol polyethylene glycol succinate together with shellac so that the present invention relates to a valvuloplasty catheter balloon coated with a coating of at least one active agent in a layer of D-α-tocopherol polyethylene glycol succinate with shellac and a top coat consisting of a polyvinyl alcohol-polyethylene glycol graft copolymer. This produces a coating which easily and quickly detaches from the valvuloplasty catheter balloon and can effectively be transferred to the vessel wall.

The valvuloplasty catheter balloon of the invention may optionally comprise further a base coat. It was surprisingly found that such a base coat is therapeutically highly useful in keeping blood vessels open, in reducing the late lumen loss and in reducing restenosis. The base coat is apparently useful by facilitating a better transfer of the active agent from the valvuloplasty catheter balloon to the vessel wall. The base coat may used for a coating with an active agent layer consisting only of the active agent or comprising the active agent and at least one polyethoxylated surfactant or a polyethoxylated emulsifier. Eventually also shellac or polyethylene glycol may be one component of this active agent containing layer.

Preferred embodiments of the invention comprise a base coat made of polyvinyl alcohol - polyethylene glycol graft copolymer, D-α-tocopherol polyethylene glycol succinate or shellac. Therefore one embodiment of the present invention relates to a valvuloplasty catheter balloon coated with at least one active agent and a top coat consisting of a polyvinyl alcohol-polyethylene glycol graft copolymer wherein the coating further comprises a base coat on said valvuloplasty catheter balloon consisting of polyvinyl alcohol-polyethylene glycol graft copolymer and/or D-α-tocopherol polyethylene glycol succinate and/or shellac. Another preferred embodiment is a valvuloplasty catheter balloon coated with at least one active agent, a matrix for the drug-coating of a valvuloplasty catheter balloon of D-α-tocopherol polyethylene glycol succinate, at least one polyethoxylated surfactant or a polyethoxylated emulsifier and a top coat consisting of a polyvinyl alcohol-polyethylene glycol graft copolymer wherein the coating further comprises a base coat of shellac or of polyvinyl alcohol - polyethylene glycol graft copolymer or a mixture of these two compounds. Another preferred embodiment is a valvuloplasty catheter balloon coated with at least one active agent a matrix for the drug-coating of a valvuloplasty catheter balloon of polyethylene glycol optionally with shellac and a top coat consisting of a polyvinyl alcohol-polyethylene glycol graft copolymer wherein the coating further comprises a base coat of shellac or of polyvinyl alcohol - polyethylene glycol graft copolymer or a mixture of these two compounds. In a particular preferred embodiment of the present invention, the valvuloplasty catheter balloon which is completely coated with the top coat, but is coated with active agent only partially, i.e. certain sections of the valvuloplasty catheter balloon, has a base coat on the complete outer surface.

A particular preferred embodiment of the present invention is a valvuloplasty catheter balloon with a coating comprising paclitaxel and a top coat consisting of a polyvinyl alcohol - polyethylene glycol graft copolymer and a base coat on said valvuloplasty catheter balloon consisting of polyvinyl alcohol - polyethylene glycol graft copolymer and/or shellac.
Further preferred is a valvuloplasty catheter balloon coated with paclitaxel, a matrix for the active agent coating of a valvuloplasty catheter balloon of D-α-tocopherol polyethylene glycol succinate, at least one polyethoxylated surfactant or a polyethoxylated emulsifier and a top coat consisting of a polyvinyl alcohol - polyethylene glycol graft copolymer, wherein the coating further comprises a base coat of shellac or of polyvinyl alcohol - polyethylene glycol graft copolymer or a mixture of these two compounds. Another preferred embodiment is a valvuloplasty catheter balloon coated with paclitaxel, a matrix for the paclitaxel coating of the valvuloplasty catheter balloon of polyethoxylated castor oil and a top coat consisting of a polyvinyl alcohol - polyethylene glycol graft copolymer wherein the coating further comprises a base coat of shellac or of polyvinyl alcohol - polyethylene glycol graft copolymer or a mixture of these two compounds. One further embodiment is a valvuloplasty catheter balloon coated with paclitaxel a matrix for the paclitaxel - coating of a valvuloplasty catheter balloon of polyethylene glycol optionally with shellac and a top coat consisting of a polyvinyl alcohol-polyethylene glycol graft copolymer wherein the coating further comprises a base coat of shellac or of polyvinyl alcohol - polyethylene glycol graft copolymer or a mixture of these two compounds. Another preferred embodiment is a valvuloplasty catheter balloon coated with paclitaxel, a matrix for the paclitaxel coating of the valvuloplasty catheter balloon of D-α-tocopherol polyethylene glycol succinate and a top coat consisting of a polyvinyl alcohol - polyethylene glycol graft copolymer wherein the coating further comprises a base coat of shellac or of polyvinyl alcohol - polyethylene glycol graft copolymer or a mixture of these two compounds.

Thus, all embodiments of the present invention comprise a top coat which is on top of the layer consisting of or comprising the active agent which is preferably paclitaxel. This top coat preferably covers completely the below layer of the active agent or containing the active agent. The top coat consists of a polyvinyl alcohol - polyethylene glycol graft copolymer and does preferably not contain any active agent and also not any polyethoxylated surfactant or polyethoxylated emulsifier and also no shellac. This special kind of top coat seems to be important to secure the coating on the valvuloplasty catheter balloon and to prevent wash off and release of the active agent, especially paclitaxel, during insertion of the to the stenotic vessel region, but also to ensure and support the transfer of the active agent to and into the vessel during dilatation. Consequently, the polyvinyl alcohol - polyethylene glycol graft copolymer top coat seems to be an essential part of the present invention.

Moreover below the top coat a coat or layer of pure active agent can be present or a coat or layer comprising the active agent together with shellac or with a polyethoxylated surfactant which is preferably polyethoxylated castor oil or with polyethylene glycol or with D-α-tocopherol polyethylene glycol succinate or together with shellac and a polyethoxylated surfactant or with polyethylene glycol and shellac or with polyethylene glycol and a polyethoxylated surfactant or with D-α-tocopherol polyethylene glycol succinate and shellac or D-α-tocopherol polyethylene glycol succinate and a polyethoxylated surfactant.

Furthermore below this top coat or layer consisting of the active agent or consisting of the active agent together with shellac, together with polyethylene glycol, together with D-α-tocopherol polyethylene glycol succinate or together with the polyethoxylated surfactant or together with shellac and the polyethoxylated surfactant or together with shellac and D-α-tocopherol polyethylene glycol succinate, a third coat or layer can be optionally present. This third coat or layer can be a base layer consisting of D-α-tocopherol polyethylene glycol succinate or of a polyethoxylated surfactant or of shellac or of a polyethoxylated surfactant and shellac. In case a polyethoxylated surfactant is used in or as the base coat and also together with the active agent in the active agent layer, preferably the same polyethoxylated surfactant is used which is preferably polyethoxylated castor oil.

Consequently the following coatings are in accordance with the present invention:
I) Balloon surface coated with paclitaxel and with polyvinyl alcohol - polyethylene glycol graft copolymers as top coat.
II) Balloon surface coated with paclitaxel together with shellac and with polyvinyl alcohol - polyethylene glycol graft copolymers as top coat.
III) Balloon surface coated with paclitaxel together with polyethoxylated castor oil and with polyvinyl alcohol - polyethylene glycol graft copolymers as top coat.
IV) Balloon surface coated with paclitaxel together with polyethylene glycol and with polyvinyl alcohol - polyethylene glycol graft copolymers as top coat.
V) Balloon surface coated with paclitaxel together with D-α-tocopherol polyethylene glycol succinate and with polyvinyl alcohol - polyethylene glycol graft copolymers as top coat.
VI) Balloon surface coated with paclitaxel together with shellac as well as polyethoxylated castor oil and with polyvinyl alcohol - polyethylene glycol graft copolymers as top coat.
VII) Balloon surface coated with paclitaxel together with shellac as well as D-α-tocopherol polyethylene glycol succinate and with polyvinyl alcohol - polyethylene glycol graft copolymers as top coat.
VIII) Balloon surface coated with paclitaxel together with a polyethoxylated surfactant as well as D-α-tocopherol polyethylene glycol succinate and with polyvinyl alcohol - polyethylene glycol graft copolymers as top coat.
IX) Balloon surface coated with paclitaxel together with shellac as well as polyethylene glycol and with polyvinyl alcohol - polyethylene glycol graft copolymers as top coat.
X) Balloon surface coated with shellac as base coat, then with paclitaxel as middle coat and with polyvinyl alcohol - polyethylene glycol graft copolymers as top coat.
XI) Balloon surface coated with shellac as base coat, then with paclitaxel together with shellac as middle coat and with polyvinyl alcohol - polyethylene glycol graft copolymers as top coat.
XII) Balloon surface coated with shellac as base coat, then with paclitaxel together with polyethoxylated castor oil as middle coat and with polyvinyl alcohol - polyethylene glycol graft copolymers as top coat.
XIII) Balloon surface coated with shellac as base coat, then with paclitaxel together with polyethylene glycol as middle coat and with polyvinyl alcohol - polyethylene glycol graft copolymers as top coat.
XIV) Balloon surface coated with shellac as base coat, then with paclitaxel together with D-α-tocopherol polyethylene glycol succinate as middle coat and with polyvinyl alcohol - polyethylene glycol graft copolymers as top coat.
XV) Balloon surface coated with shellac as base coat, then with paclitaxel together with shellac as well as D-α-tocopherol polyethylene glycol succinate as middle coat and with polyvinyl alcohol - polyethylene glycol graft copolymers as top coat.
XVI) Balloon surface coated with shellac as base coat, then with paclitaxel together with D-α-tocopherol polyethylene glycol succinate as well as polyethoxylated castor oil as middle coat and with polyvinyl alcohol - polyethylene glycol graft copolymers as top coat.
XVII) Balloon surface coated with shellac as base coat, then with paclitaxel together with shellac as well as polyethoxylated castor oil as middle coat and with polyvinyl alcohol - polyethylene glycol graft copolymers as top coat.
XVIII) Balloon surface coated with shellac as base coat, then with paclitaxel together with shellac as well as polyethylene glycol as middle coat and with polyvinyl alcohol - polyethylene glycol graft copolymers as top coat.
XIX) Balloon surface coated with polyethoxylated castor oil as base coat, then with paclitaxel as middle coat and with polyvinyl alcohol - polyethylene glycol graft copolymers as top coat.
XX) Balloon surface coated with polyethoxylated castor oil as base coat, then with paclitaxel together with shellac as middle coat and with polyvinyl alcohol - polyethylene glycol graft copolymers as top coat.
XXI) Balloon surface coated with polyethoxylated castor oil as base coat, then with paclitaxel together with polyethoxylated castor oil as middle coat and with polyvinyl alcohol - polyethylene glycol graft copolymers as top coat.
XXII) Balloon surface coated with polyethoxylated castor oil as base coat, then with paclitaxel together with shellac as well as polyethoxylated castor oil as middle coat and with polyvinyl alcohol - polyethylene glycol graft copolymers as top coat.
XXIII) Balloon surface coated with shellac and polyethoxylated castor oil as base coat, then with paclitaxel as middle coat and with polyvinyl alcohol - polyethylene glycol graft copolymers as top coat.
XXIV) Balloon surface coated with shellac and polyethoxylated castor oil as base coat, then with paclitaxel together with shellac as middle coat and with polyvinyl alcohol - polyethylene glycol graft copolymers as top coat.
XXV) Balloon surface coated with shellac and polyethoxylated castor oil as base coat, then with paclitaxel together with polyethoxylated castor oil as middle coat and with polyvinyl alcohol - polyethylene glycol graft copolymers as top coat.
XXVI) Balloon surface coated with shellac and polyethoxylated castor oil as base coat, then with paclitaxel together with shellac as well as polyethoxylated castor oil as middle coat and with polyvinyl alcohol - polyethylene glycol graft copolymers as top coat.
XXVII) Balloon surface coated with shellac and polyethoxylated castor oil as base coat, then with paclitaxel together with shellac as well as polyethylene glycol oil as middle coat and with polyvinyl alcohol - polyethylene glycol graft copolymers as top coat.

Or in other words:
I) First layer paclitaxel and top coat polyvinyl alcohol - polyethylene glycol graft copolymer.
II) First layer paclitaxel together with shellac and top coat polyvinyl alcohol - polyethylene glycol graft copolymer.
III) First layer paclitaxel together with polyethoxylated castor oil and top coat polyvinyl alcohol - polyethylene glycol graft copolymer.
IV) First layer paclitaxel together with polyethylene glycol and top coat polyvinyl alcohol - polyethylene glycol graft copolymer.
V) First layer paclitaxel together with shellac as well as polyethoxylated castor oil and top coat polyvinyl alcohol - polyethylene glycol graft copolymer.
VI) First layer paclitaxel together with shellac as well as polyethylene glycol and top coat polyvinyl alcohol - polyethylene glycol graft copolymer.
VII) First layer paclitaxel together with D-α-tocopherol polyethylene glycol succinate and top coat polyvinyl alcohol - polyethylene glycol graft copolymer.
VIII) First layer paclitaxel together with shellac as well as D-α-tocopherol polyethylene glycol succinate and top coat polyvinyl alcohol - polyethylene glycol graft copolymer.
IX) First layer paclitaxel together with polyethoxylated castor oil as well as D-α-tocopherol polyethylene glycol succinate and top coat polyvinyl alcohol - polyethylene glycol graft copolymer.
X) Base coat shellac, middle coat paclitaxel, top coat polyvinyl alcohol - polyethylene glycol graft copolymer.
XI) Base coat shellac, middle coat paclitaxel together with shellac, top coat polyvinyl alcohol - polyethylene glycol graft copolymer.
XII) Base coat shellac, middle coat paclitaxel together with D-α-tocopherol polyethylene glycol succinate, top coat polyvinyl alcohol - polyethylene glycol graft copolymer.
XIII) Base coat shellac, middle coat paclitaxel together with polyethoxylated castor oil, top coat polyvinyl alcohol - polyethylene glycol graft copolymer.
XIV) Base coat shellac, middle coat paclitaxel together with polyethylene glycol, top coat polyvinyl alcohol - polyethylene glycol graft copolymer.
XV) Base coat shellac, middle coat paclitaxel together with shellac as well as polyethoxylated castor oil, top coat polyvinyl alcohol - polyethylene glycol graft copolymer.
XVI) Base coat shellac, middle coat paclitaxel together with shellac as well as D-α-tocopherol polyethylene glycol succinate, top coat polyvinyl alcohol - polyethylene glycol graft copolymer.
XVII) Base coat shellac, middle coat paclitaxel together with D-α-tocopherol polyethylene glycol succinate as well as polyethoxylated castor oil, top coat polyvinyl alcohol - polyethylene glycol graft copolymer.
XVIII) Base coat polyethoxylated castor oil, middle coat paclitaxel, top coat polyvinyl alcohol - polyethylene glycol graft copolymer.
XIX) Base coat polyethoxylated castor oil, middle coat paclitaxel together with shellac, top coat polyvinyl alcohol - polyethylene glycol graft copolymer.
XX) Base coat polyethoxylated castor oil, middle coat paclitaxel together with polyethylene glycol, top coat polyvinyl alcohol - polyethylene glycol graft copolymer.
XXI) Base coat polyethoxylated castor oil, middle coat paclitaxel together with polyethoxylated castor oil, top coat polyvinyl alcohol - polyethylene glycol graft copolymer.
XXII) Base coat polyethoxylated castor oil, middle coat paclitaxel together with shellac as well as polyethoxylated castor oil, top coat polyvinyl alcohol - polyethylene glycol graft copolymer.
XXIII) Base coat shellac and polyethoxylated castor oil, middle coat paclitaxel, top coat polyvinyl alcohol - polyethylene glycol graft copolymer.
XXIV) Base coat shellac and polyethoxylated castor oil, middle coat paclitaxel together with shellac, top coat polyvinyl alcohol - polyethylene glycol graft copolymer.
XXV) Base coat shellac and polyethoxylated castor oil, middle coat paclitaxel together with polyethylene glycol, top coat polyvinyl alcohol - polyethylene glycol graft copolymer.
XXVI) Base coat shellac and polyethoxylated castor oil, middle coat paclitaxel together with polyethoxylated castor oil, top coat polyvinyl alcohol - polyethylene glycol graft copolymer.
XXVII) Base coat shellac and polyethoxylated castor oil, middle coat paclitaxel together with shellac as well as polyethoxylated castor oil, top coat polyvinyl alcohol - polyethylene glycol graft copolymer.
XXVIII) Base coat shellac and polyethoxylated castor oil, middle coat paclitaxel together with shellac as well as polyethylene glycol, top coat polyvinyl alcohol - polyethylene glycol graft copolymer.

Shellac is a natural resin produced from the glandular secretion of a number of species of lac-producing insects. Lac insects belong to the order of Hemiptera, superfamily Coccoidea such as Metatachardia, Laccifer, Tachordiella, and others, however, members of two families - Lacciferidae and Tachardinidae are more prominent in lac secretion. The one that is commercially cultured is Kerria lacca, which is also known by such synonyms as Laccifer lacca Ker, Tachardia lacca, and Carteria lacca. Kerria lacca is an Indian scale insect, which infests branches of numerous trees from the East Indies, such as Butea frondos Rosch, Acacia arabica Willd and Ficus religiosa Linn. Shellac is the only commercially used natural resin of animal origin and is quite different from all other natural resins. More recently, as a new awareness about the environments and the toxicity of chemical raw-material is noticeable everywhere, shellac or shellac modified resin are gaining importance due to their interesting and unique characteristics. Broken branches are sold as stick lac and, after grounding and washing with water to eliminate wood and red pigments (lac dye), seed lac is obtained. Purification of seed lac gives the more homogeneous product known as shellac. Its use in Europe began towards the end of the 16^{th} century mainly as a varnish (mostly known as "French polish") for wooden objects, musical instruments and gilding, as a protective for vinyl disks and mural paintings, as an insulating material for earlier radios and other electrical tools and as an adhesive in the restoration of pottery.

Raw material shellac consists of 70-80% resin, 4-8% dye, 6-7% hard and high gloss finished wax, 3% water, up to 9% vegetable and animal impurities and aroma substances. Shellac resin is a complicated mixture of aliphatic (60%) and sesquiterpenoid acids (32%) and their esters. Sesquiterpenoid acids are jalaric and laccijalaric acids (structure I and II) and aliphatic acids are aleuritic (III) and butolic acid.

A possibility for chemical description of resin molecule is a structure model where in each case 4 molecules jalaric or laccijalaric acid and aleuritic acid are connected by ester bonding alternately.

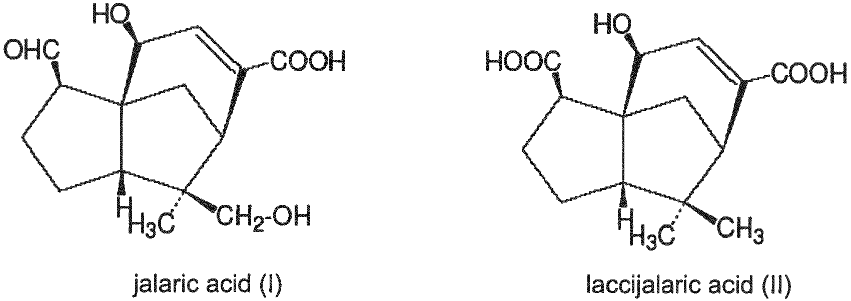

Its chemical composition is almost constant, although the amount of some components changes depending on the nature of host trees on which the insects grows. By Cannizzaro-type disproportionation under alkaline hydrolysis will be synthesized from these acids shellolic acid (IV) and deviate compounds. Purified shellac consists of two main components. These components are 9,10,16-trihydroxypalmitic acid (aleuritic acid) CAS [53-387-9] and shellolic acid (IV).

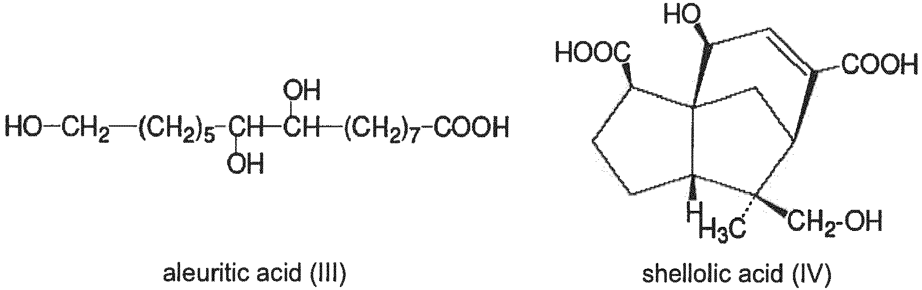

A modification with other natural or synthetic resins or co-polymerization with various monomers is possible to cross link shellac, modified shellac resins and shellac copolymers with urea, melamine, formaldehyde, isocyanides, other chemical processes like polymerization, hydroxylation, extrication, etc. are possible.

Followings are the commercial grades of shellac:
- Seedlac - Dewaxed Shellac
- Hand Made Shellac - Dewaxed Bleached Shellac
- Machine Made Shellac - Aleuritic Acid

Major Properties of shellac are:
- Shellac is a hard natural resin
- Shellac has a good resistance against solvent
- Shellac based on hydrocarbons
- Shellac is non toxic
- Shellac is thermoplastic
- Shellac is physiologically harmless
- Shellac is approved for various applications in the food industry.
- Shellac is not UV-resistant
- Shellac is soluble in lower alcohol's
- Shellac has excellent dielectric properties high dielectric strength, low dielectric constant, good tracking resistance etc.
- Shellac has a low melting point (65-85°C).
- Shellac is water soluble in water-alkaline solutions
- Coatings do not change their electric properties under UV-radiation.
- Shellac has excellent film forming properties.
- Shellac has low thermal conductivity and a low coefficient of expansion forms smooth, high gloss films and surfaces.
Shellac coating has excellent adhesion to many coatings and can be polished.

A possibility for chemical description of resin molecule is a structure model where in each case 4 molecules jalaric or laccijalaric acid and aleuritic acid are connected by ester bonding alternately.

D-α-tocopherol polyethylene glycol succinate is prepared by the esterification of polyethylene glycol 1000 to the acid group of crystalline D-alpha tocopheryl acid succinate. Common synonyms are TPGS, Vitamin E polyethylene glycol succinate and Vitamin E-TPGS. The CAS Number is 9002-96-4. D-α-tocopherol polyethylene glycol succinate is water soluble. D-alpha tocopheryl polyethylene glycol 1000 succinate is a synthetic amphiphile, can therefore act as a surfactant and is a potent instrument in the creation of lipophilic and poorly soluble compounds and greatly enhances the absorption, bioavailability and effectiveness of pharmaceutical ingredients.

Its chemical structure is as follows:

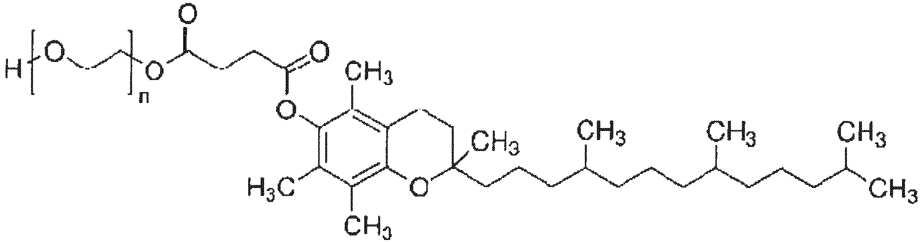

The term "valvuloplasty" as used herein refers to the widening of a stenotic valve using a balloon catheter also called valvotomy. Preferred are aortic valvuloplasty in repair of a stenotic aortic valve and mitral valvuloplasty in the correction of an uncomplicated mitral valve stenosis. The active agent on the valvuloplasty catheter balloon is delivered from the external surface of the balloon to the valve tissues during the inflation of the balloon in performing valvuloplasty.

The term "base coat" as used herein refers to a layer of the coating of a valvuloplasty catheter balloon which is immediately on the surface of the valvuloplasty catheter balloon. This layer is a first layer which directly overlays the material of the valvuloplasty catheter balloon as a priming coat which mainly increases the adherence of the active agent containing layer. The term "top layer" or "top coat" as used herein refers to a layer of the balloon coating free of an active agent which overlays the active agent containing layer.

The term "uncoated" as used herein refers to a valvuloplasty catheter balloon with a smooth or structured or roughened surface without any active agent coating, i.e. the balloon surface does not comprise a pharmaceutically active agent and especially no antiproliferative, immunosuppressive, anti-angiogenic, anti-inflammatory, anti-restenotic, or anti-thrombotic agent and no coating containing an antiproliferative, immunosuppressive, anti-angiogenic, anti-inflammatory, anti-restenotic, and/or anti-thrombotic agent.

The present invention refers to a valvuloplasty catheter balloon coated with at least one active agent and a top coat on said active agent coat or layer consisting of a polyvinyl alcohol - polyethylene glycol graft copolymer. Materials used for the balloon of the valvuloplasty catheter are such materials which are commonly used, wherein the following polymers are particularly preferred: polyamides, block copolymers of polyamide, polyether and polyester, polyurethanes, polyesters and polyolefins.

The valvuloplasty catheter balloon of the invention is dilatable or expandable and is most preferably an aortic valvuloplasty catheter balloon. Such catheters or catheter balloons having a coating according to the invention are preferably used for treating heart valve stenosis like the mitral stenosis or the aortic stenosis and for prophylaxis of restenosis in aortic or mitral valve stenosis.

Aortic valvuloplasty balloons were introduced in the second half of the 70s. Hence, today there are many manufactures distributing valvuloplasty catheter balloons having different shapes. In regard to the present invention all common valvuloplasty balloons may be used for coating.

More preferably the valvuloplasty catheter balloon is only coated in its middle part which comes into contact with the heart valve(s). The term "middle segment" refers to the part of the surface of the catheter balloon which comes into contact or which can come into contact with tissue or a heart valve. Thus a preferred embodiment of the present invention relates to valvuloplasty catheter balloons which are partly coated with the inventive coating or the layer of the inventive coating containing the active agent is partly coated theron.

The middle segment may be the central section of a balloon especially for mitral valvuloplasty which is inflated after the distal and proximal segment. The middle segment can also be each structure of the valvuloplasty balloon formed to have a greater or an adapted contact surface for the valve. Another possibility is the waist of a balloon having a glass hour shape or a fold or wing of the balloon surrounding the valves.

This partly coating saves costs and coating material and reduces exposure of the patient with additional pharmaceutically active agent which is present on parts of the catheter balloon which do not come into contact with tissue or heart valves. Thus a preferred embodiment of the present invention relates to valvuloplasty catheter balloons which are partly coated with the active agent. Such balloons are provided with folds or wings forming mainly closed cavities if the balloon is in its deflated or compressed state. Upon expansion by inflation of the balloon the folds or wings bend outward and are capable of immediate release of substances contained within the folds or pressing said substances against the valves, respectively, upon contact with the valves.

Such balloons are advantageous since the substances enclosed in the folds or respectively paclitaxel enclosed in the folds are protected from being detached too soon during the insertion of the catheter. The surface of the valvuloplasty catheter balloon may be textured, smooth, rough, harsh, provided with cavities or provided with channels open towards the outside of the balloon. In the case, a textured surface of the valvuloplasty catheter balloon is desired the surface of the valvuloplasty catheter balloon can be textured mechanically, chemically, electronically and/or by means of radiation to allow for an improved adhesion of the active agent and to assist the precipitation or crystallization of the active agent. It is of importance to avoid all damage to the valvuloplasty catheter balloons while the balloon surface is textured and to ensure that their capability to expand is not disadvantageously affected. Thus, the methods for micro texturing the valvuloplasty balloon surface must not lead to the formation of holes, micropores or fissures in the balloon material. Ideally, only the outer surface of the balloon, i.e. to a maximum depth of 1 µm, is textured.

The inventive valvuloplasty catheter balloon can be used within a scoring catheter, also called a cutting or blade catheter. A scoring catheter comprises a catheter body having a proximal end and a distal end, an valvuloplasty catheter balloon near the distal end of the catheter body, and a, mostly non-axial, scoring structure carried over the balloon. The scoring structure will be able to score or cut stenotic material along lines. For example, the scoring lines may be helical, serpentine, zig-zag, or may combine some axial components together with non-axial components. Such a scoring catheter or cutting catheter has at least one structure, like recesses, metallic nets or small blades mounted over the balloon for generating micro-cracks in the plaque, i.e. calcified deposits, during expansion.

One embodiment of the invention is a valvuloplasty catheter balloon coated with at least one active agent and a top coat consisting of a polyvinyl alcohol - polyethylene glycol graft copolymer. Preferred embodiments of the invention have a coating comprising at least one polyethoxylated surfactant or at least one polyethoxylated emulsifier. The at least one polyethoxylated surfactant or at least one polyethoxylated emulsifier may be applied to the valvuloplasty catheter balloon either before the active agent is applied or the at least one polyethoxylated surfactant or at least one polyethoxylated emulsifier is applied together with the active agent, which means that one coating solution comprises the active agent and the at least one polyethoxylated surfactant or at least one polyethoxylated emulsifier. Therefore one embodiment of the invention is a valvuloplasty catheter balloon coated with at least one active agent in a matrix comprising at least one polyethoxylated surfactant or at least one polyethoxylated emulsifier. The term matrix is used in this application for a compound the active agent is embedded in or incorporated in.

Other embodiments of the invention have a coating comprising D-α-tocopheryl polyethylene glycol succinate. The D-α-tocopherol polyethylene glycol succinate may be applied to the valvuloplasty catheter balloon either before the active agent is applied or the D-α-tocopherol polyethylene glycol succinate is applied together with the active agent, which means that one coating solution comprises the active agent and the D-α-tocopherol polyethylene glycol succinate. Therefore one embodiment of the invention is a valvuloplasty catheter balloon coated with at least one active agent in a matrix comprising D-α-tocopherol polyethylene glycol succinate.

The term "emulsifier" as used herein is a substance that stabilizes an emulsion by increasing its kinetic stability. An emulsion is a mixture of two or more liquids which are normally immiscible. With other words, emulsifiers are excipients or additives, which serve for mixing two liquids which may not be mixed with each other in a so-called emulsion and to stabilize the latter. Similar to emulsifiers are so called "surfactants". The term as used herein refers to compounds that lower the surface tension of a liquid, the interfacial tension between two liquids, or that between a liquid and a solid. Surfactants may act as detergents, wetting agents, emulsifiers, foaming agents, and dispersants. Emulsifiers and surfactants, respectively, are usually organic compounds that are amphiphilic, meaning they contain both hydrophobic groups and hydrophilic groups. Consequently an amphiphilic molecule contains both a water insoluble component and a water soluble component. Emulsifiers and surfactants as used herein are classified according to the composition of their hydrophilic part. Emulsifiers and surfactants, respectively, suitable for the present invention are aromatic hydrocarbons, alkanes, alkenes, cycloalkanes, alkyne-based hydrocarbons, fluorosurfactants such as perfluorooctanesulfonic acid, perfluorooctanoic acid, perfluorononanoic acid and siloxane being polyethoxylated. Ethoxylation is a chemical process in which ethylene oxide is added to alcohols and phenols to generate surfactants and emulsifiers. Consequently polyethoxylation is a chemical process wherein more than one group of the respective alcohol or phenol are ethoxylated. D-α-tocopherol polyethylene glycol succinate and polyethoxylated compounds are preferred emulsifiers and surfactants, respectively, in the present invention; more preferred are polyethoxylated surfactants or polyethoxylated emulsifiers selected from the group consisting of or comprising: polyethoxylated alcohols, polyethoxylated oils, polyethoxylated castor oil, polyethoxylated glycerol, polyethoxylated fatty acid esters, polyethoxylated phenols, polyethoxylated amines and polyethoxylated fatty alcohols. Among these surfactants or emulsifiers polyethoxylated castor oils are more preferred. Further preferred are compounds which are produced by reacting higher saturated fatty alcohols with ethylene oxide, and particularly preferred are compounds which are made by reacting castor oil with ethylene oxide in a ration of 1:35, which means that it is prepared by reacting 35 moles of ethylene oxide with each mol of castor oil. Thereby the hydroxyl-groups of the castor oil triglyceride have ethoxylated with ethylene oxide to from polyethylene glycol ethers. This is followed by a purification process with regard to water content, potassium ions and free fatty acids, particularly ricinoleic, oleic and palmitic acids. The compound received is a white to yellowish paste or cloudy liquid. On heating, the last solid constituents melt at 26°C to yield a clear oily liquid with a weak but characteristic odour. The HLB (hydrophilic-lipophilic balance) value lies between 12 and 14. The critical micelle concentration (CMC) lies at approx. 0.02%. Said preferred polyethoxylated castor oil is also named macrogolglycerol ricinoleate Ph.Eur., Polyoxyl-35-castor oil USP/NF and is distributed under the trademark Cremophor^{®} ELP by BASF.

Further components for improving the emulsifying properties of the latter two components can be polyethylene glycol esters of ricinoleic acid, polyethylene glycols and polyethylene glycol esters of glycerol.

Polyethoxylated fatty alcohols and polyethoxylated castor oil are dissolved in water and alcohol to form either a colloid or a clear solution. The compounds are soluble in vegetable and mineral oils and fats. The warm emulsifiers can be mixed with mineral, vegetable and synthetic fats and oils, as well as with fatty alcohols, fatty acids, mono- and di-stearates, and polyethylene glycols. In aqueous solution, these compounds are largely resistant to acids, bases and salts. The presence of these electrolytes does not impair the product's efficiency as emulsifying agent. The particularly preferred polyethoxylated castor oil dissolves in a wide range of further organic solvents, such as ethanol, n-propanol, isopropanol, ethyl acetate, chloroform, carbon tetrachloride, trichloroethylene, toluene and xylene.

One embodiment of the invention is a valvuloplasty catheter balloon coated with at least one active agent and a top coat consisting of a polyvinyl alcohol - polyethylene glycol graft copolymer. Preferred embodiments of the invention have a coating comprising further polyethylene glycol. According to the invention polyethylene glycol may be applied to the balloon surface as a matrix substance for the at least one active agent. Polyethylene glycol may be used alone or together with shellac or together with the at least one polyethoxylated surfactant.

Antiproliferative, immunosuppressive, anti-angiogenic, anti-inflammatory, anti-restenotic and/or anti-thrombotic agent are used as active agents. The active agents are used individually or combined having same or different concentration. These active agents can be applied to the surface of the valvuloplasty catheter balloon forming a pure active agent layer without any matrix but being at least covered by the top coat of the invention. It is also possible, that the active agent is applied being dissolved, emulsified, suspended or dispersed in the at least one polyethoxylated surfactant or polyethoxylated emulsifier and/or shellac and/or polyethylene glycol. Such an inclusion of the active agents ensures that a short-term and controlled release of the active agents from the matrix takes place by the balloon dilatation during valvuloplasty. Further, there is the possibility, that the active agent or the combination of active agents is applied to the surface after coating of the valvuloplasty catheter balloon with the at least one polyethoxylated surfactant or polyethoxylated emulsifier and/or shellac and/or polyethylene glycol and is soaked into this layer on the surface of the valvuloplasty catheter balloon.

Preferred is a coated valvuloplasty catheter balloon, wherein the active agent is an antiproliferative, immunosuppressive, anti-angiogenic, anti-inflammatory, anti-restenotic and/or anti-thrombotic agent. It is further preferred if the active agent is selected from the group consisting of or comprising:
abciximab, acemetacin, acetylvismione B, aclarubicin, ademetionine, adriamycin, aescin, afromosone, akagerine, aldesleukin, amidorone, aminoglutethimide, amsacrine, anakinra, anastrozole, anemonin, anopterine, antimycotics antithrombotics, apocymarin, argatroban, aristolactam-All, aristolochic acid, ascomycin, asparaginase, aspirin, atorvastatin, auranofin, azathioprine, azithromycin, baccatin, bafilomycin, basiliximab, bendamustine, benzocaine, berberine, betulin, betulinic acid, bilobol, bisparthenolidine, bleomycin, combrestatin, Boswellic acids, bruceanol A, B and C, bryophyllin A, busulfan, antithrombin, bivalirudin, cadherins, camptothecin, capecitabine, o-carbamoyl-phenoxyacetic acid, carboplatin, carmustine, celecoxib, cepharanthin, cerivastatin, CETP inhibitors, chlorambucil, chloroquine phosphate, cicutoxin, ciprofloxacin, cisplatin, cladribine, clarithromycin, colchicine, concanamycin, coumadin, C-type natriuretic peptide (CNP), cudraisoflavone A, curcumin, cyclophosphamide, ciclosporin A, cytarabine, dacarbazine, daclizumab, dactinomycin, dapsone, daunorubicin, diclofenac, 1,11-dimethoxycanthin-6-one, docetaxel, doxorubicin, daunamycin, epirubicin, erythromycin, estramustine, etoposide, filgrastim, fluroblastin, fluvastatin, fludarabine, fludarabine-5'-dihydrogen phosphate, fluorouracil, folimycin, fosfestrol, gemcitabine, ghalakinoside, ginkgol, ginkgolic acid, glycoside 1a, 4-hydroxyoxycyclo phosphamide, idarubicin, ifosfamide, josamycin, lapachol, lomustine, lovastatin, melphalan, midecamycin, mitoxantrone, nimustine, pitavastatin, pravastatin, procarbazine, mitomycin, methotrexate, mercaptopurine, thioguanine, oxaliplatin, irinotecan, topotecan, hydroxycarbamide, miltefosine, pentostatin, pegaspargase, exemestane, letrozole, formestane, mycophenolate mofetil, β-lapachone, podophyllotoxin, podophyllic acid-2-ethyl hydrazide, molgramostim (rhuGM-CSF), peginterferon α-2b, lenograstim (r-HuG-CSF), macrogol, selectin (cytokine antagonist), cytokinin inhibitors, COX-2 inhibitor, angiopeptin, monoclonal antibodies inhibiting muscle cell proliferation, bFGF antagonists, probucol, prostaglandins, 1-hydroxy-11-methoxycanthin-6-one, scopoletin, NO donors, pentaerythrityl tetranitrate and sydnoimines, tamoxifen, staurosporine, β-estradiol, α-estradiol, estriol, estrone, ethinyl estradiol, medroxyprogesterone, estradiol cypionates, estradiol benzoates, tranilast, kamebakaurin and other terpenoids used in cancer therapy, verapamil, tyrosine kinase inhibitors (tyrphostins), paclitaxel, 6-α-hydroxy-paclitaxel, taxoteres, mofebutazone, lonazolac, lidocaine, ketoprofen, mefenamic acid, piroxicam, meloxicam, penicillamine, hydroxychloroquine, sodium aurothiomalate, oxaceprol, β-sitosterol, myrtecaine, polidocanol, nonivamide, levomenthol, ellipticine, D-24851 (Calbiochem), colcemid, cytochalasin A-E, indanocine, nocodazole, bacitracin, vitronectin receptor antagonists, azelastine, guanidyl cyclase stimulator, tissue inhibitor of metal proteinase-1 and -2, free nucleic acids, nucleic acids incorporated into virus transmitters, DNA and RNA fragments, plasminogen activator inhibitor 1, plasminogen activator inhibitor 2, antisense oligonucleotides, VEGF inhibitors, IGF-1, active agents from the group of antibiotics, cefadroxil, cefazolin, cefaclor, cefoxitin, tobramycin, gentamicin, penicillins, dicloxacillin, oxacillin, sulfonamides, metronidazole, enoxaparin, heparin, hirudin, PPACK, protamine, prourokinase, streptokinase, warfarin, urokinase, vasodilators, dipyramidole, trapidil, nitroprussides, PDGF antagonists, triazolopyrimidine, seramin, ACE inhibitors, captopril, cilazapril, lisinopril, enalapril, losartan, thioprotease inhibitors, prostacyclin, vapiprost, interferon α, β and γ, histamine antagonists, serotonin blockers, apoptosis inhibitors, apoptosis regulators, halofuginone, nifedipine, tocopherol, tranilast, molsidomine, tea polyphenols, epicatechin gallate, epigallocatechin gallate, leflunomide, etanercept, sulfasalazine, tetracycline, triamcinolone, mutamycin, procainimide, retinoic acid, quinidine, disopyrimide, flecainide, propafenone, sotalol, natural and synthetically obtained steroids such as bryophyllin A, inotodiol, maquiroside A, ghalakinoside, mansonine, strebloside, hydrocortisone, betamethasone, dexamethasone, non-steroidal substances (NSAIDS), fenoprofen, ibuprofen, indomethacin, naproxen, phenylbutazone, antiviral agents, acyclovir, ganciclovir zidovudine, clotrimazole, flucytosine, griseofulvin, ketoconazole, miconazole, nystatin, terbinafine, antiprotozoal agents, chloroquine, mefloquine, quinine, natural terpenoids, hippocaesculin, barringtogenol-C21-angelate, 14-dehydroagrostistachin, agroskerin, agrostistachin, 17-hydroxyagrostistachin, ovatodiolids, 4,7-oxycycloanisomelic acid baccharinoids B1, B2, B3 and B7, tubeimoside, bruceantinoside C, yadanziosides N and P, isodeoxyelephantopin, tomenphantopin A and B, coronarin A,B C and D, ursolic acid, hyptatic acid A, iso-iridogermanal, maytenfoliol, effusantin A, excisanin A and B, longikaurin B, sculponeatin C, kamebaunin, leukamenin A and B, 13,18-dehydro-6-alpha-senecioyloxychaparrin, taxamairin A and B, regenilol, triptolide, cymarin, hydroxyanopterine, protoanemonin, cheliburin chloride, sinococuline A and B, dihydronitidine, nitidine chloride, 12-β-hydroxypregnadien-3,20-dione, helenalin, indicine, indicine-N-oxide, lasiocarpine, inotodiol, podophyllotoxin, justicidin A and B, larreatin, malloterin, mallotochromanol, isobutyrylmallotochromanol, marchantin A, maytansin, lycoridicin, margetine, pancratistatin, liriodenine, oxoushinsunine, periplocoside A, deoxypsorospermin, psychorubin, ricin A, sanguinarine, manwu wheat acid, methylsorbifolin, chromones of spathelia, stizophyllin, dihydrousambaraensine, hydroxyusambarine, strychnopentamine, strychnophylline, usambarine, usambarensine, liriodenine, daphnoretin, lariciresinol, methoxylariciresinol, syringaresinol, sirolimus (rapamycin), biolimus A9, pimecrolimus, everolimus, myolimus, novolimus, ridaforolimus, temsirolimus, zotarolimus, tacrolimus, fasudil, epothilones, somatostatin, roxithromycin, troleandomycin, simvastatin, rosuvastatin, vinblastine, vincristine, vindesine, teniposide, vinorelbine, trofosfamide, treosulfan, temozolomide, thiotepa, tretinoin, spiramycin, umbelliferone, desacetylvismione A, vismione A and B, zeorin.

In a preferred embodiment of the invention, a second active agent may be added to the active agent containing layer, wherein the second active agent is selected from the same group of compounds listed in the previous paragraph.

The preferred solvents for the active agents are volatile easily removable solvents such as acetone, ethyl acetate, ethanol, methanol, DMSO (dimethyl sulfoxide), THF (tetrahydrofurane), chloroform, methylene chloride.

More preferred is that the active agent of the present invention is selected from the group comprising or consisting of: paclitaxel, taxanes, docetaxel, rapamycin, sirolimus (rapamycin), rapamycin, biolimus A9, pimecrolimus, everolimus, myolimus, novolimus, ridaforolimus, temsirolimus, zotarolimus, tacrolimus, fasudil and epothilones.

The particularly preferred active agent in the present invention is paclitaxel. Paclitaxel is commercially available from several suppliers. Paclitaxel is known under the trademark name of Taxol^{®} and is also designated with various synonymous names such as: BMS 181339-01, BMS-181339, BMS-181339-01, Capxol, DRG-0190, DTS-301, Ebetaxel, Genaxol, Genexol, Genexol-PM, HSDB 6839, Intaxel, KBio2_002509, KBio2_005077, KBio2_007645, KBio3_002987, KBioGR_002509, KBioSS_002517, LipoPac, MBT 0206, MPI-5018, Nanotaxel, NC160-000601, Nova-12005, NSC 125973, NSC-125973, NSC125973, Onxol, Pacligel, Paxceed, Paxene, Paxoral, Plaxicel, QW 8184, SDP-013, TA1, Tax-11-en-9-on, TaxAlbin, Taxol A, Xorane or Yewtaxan.

Its chemical structure is as follows:

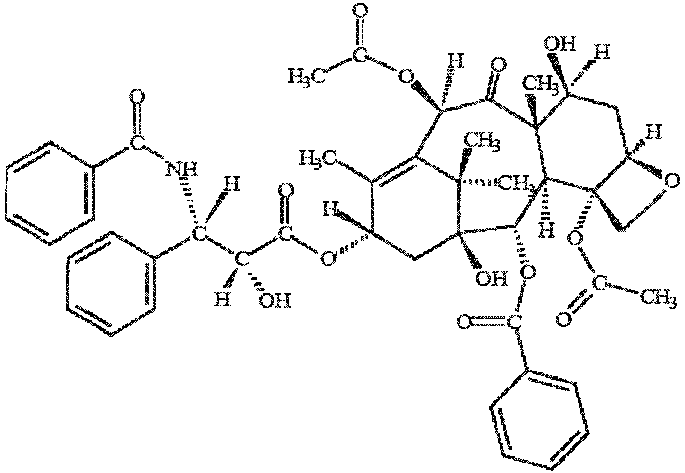

IUPAC nomenclature is: [2aR-[2a,4,4a,6,9(R*,S*),11,12,12a,12b]]-(benzoylamino)-hydroxybenzene propionic acid 6,12b-bis-(acetyloxy)-12-(benzoyloxy)-2a-3, 4, 4a, 5, 6, 9, 10, 11, 12, 12a, 12b-dodecahydro-4,11-dihydroxy-4a,8,13,13-tetramethyl-5-oxo-7,11-methano-1H-cyclodeca[3,4]benz[1,2-b]oxet-9-yl ester).

Paclitaxel is highly soluble in dimethyl sulfoxide (DMSO) and methanol as well as in anhydrous ethanol, but is comparatively poorly soluble in water. Paclitaxel is especially stable at a pH between 3 and 5 and can be stored for long periods, whereas it is comparatively instable at alkaline pH.
Dimethyl sulfoxide (DMSO), acetone, ethyl acetate, ethanol and methanol are used as a solvent for paclitaxel.

The microtubule-stabilising agent paclitaxel exerts not only anti-proliferative and therefore anti-restenotic effects but is also a significant anti-inflammatory agent. Paclitaxel is therefore a very prosperous active agent not only for the prophylaxis of restenosis but also for prophylaxis of endocarditis, one common unwanted side effect of valvuloplasty. Further this anti-inflammatory activity makes paclitaxel especially suitable for the treatment of valve restenosis of patients suffering from a rheumatic, underlying disease or a valve stenosis cause by rheumatic fever (rheumatic aortic stenosis).

One especially preferred embodiment is therefore a valvuloplasty catheter balloon of a balloon catheter coated with one layer comprising at least one polyethoxylated castor oil, paclitaxel and shellac. Further the coating of said valvuloplasty catheter balloon comprises a top coat or second layer of polyvinyl alcohol-polyethylene glycol graft copolymer. Preferably paclitaxel and the polyethoxylated castor oil are used in 1.0 weight equivalents to 0.10 to 1.2 weight equivalents.

Another especially preferred embodiment is further a valvuloplasty catheter balloon of a balloon catheter coated with one layer comprising D-α-tocopherol polyethylene glycol succinate, paclitaxel and/or shellac. Further the coating of said valvuloplasty catheter balloon comprises a top coat or second layer of polyvinyl alcohol-polyethylene glycol graft copolymer.

As a very prosperous active agent for the same purpose of restenosis prophylaxis rapamycin (syn. sirolimus) a hydrophilic macrolid antibiotic appears. This active agent is especially utilized in transplantation medicine as immunosuppressive, wherein contrary to other immunosuppressive active agents rapamycin also inhibits tumor formation. As after transplantation an increased risk of tumor formation exists for the patient the administration of rapamycin is advantageous because other immunosuppressives such as cyclosporin A can even promote tumor formation as is known. Its chemical structure is as follows:

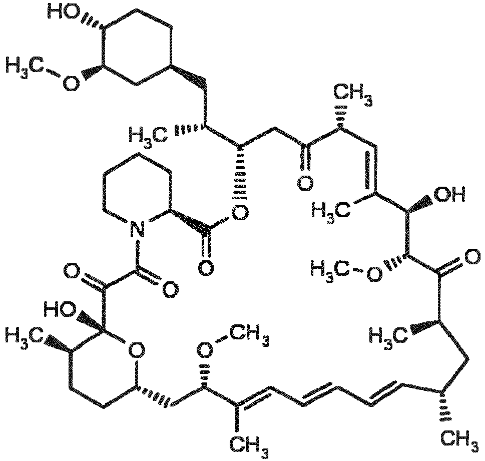

### IUPAC name:

[3S-[3R*[E(1S*,3S*,4S*)],4S*,5R*,8S*,9E,12R*,14R*,15S*,16R*,18S*,19S*,26aR*]]-5,6,8,11,12,13,14,15,16,17,18,19,24,25,26,26a-hexadecahydro-5,19-dihydroxy-3-[2-(4-hydroxy-3-methoxycyclohexyl)-1-methylethenyl]-14,16-dimethoxy-4,10,12,18-tetramethyl-8-(2-propenyl)-15,19-epoxy-3H-pyrido[2,1-c][1,4]-oxaazacyclotricosine-1,7,20, 21(4H,23H)-tetron monohydrate.

Rapamycin's mechanism of action is not yet known in detail but it is attributed especially to the complex formation with the protein mTOR (mammalian target of rapamycin) a phosphatidylinositol-3 kinase of 282kD. As mTOR is responsible for a series of cytokin-mediated signal transduction paths i.a. also for signal paths which are necessary for cell division besides the immunosuppressive effect, rapamycin or sirolimus has also antiphlogistic, antiproliferative and even antimycotic properties.

Proliferation is interrupted in the late G1 phase by stopping the ribosomal protein synthesis. Compared to other antiproliferative active agents rapamycin's mechanism of action can be pointed out as special likewise paclitaxel but which is strongly hydrophobic. Moreover, the immunosuppressive and antiphlogistic effects as described above are more than advantageous because also the extent of inflammatory reactions and of the total immune response as their premature control after stent implantation is decisive for the further success.

Thus, rapamycin has all of the necessary conditions for the utilization against stenoses and restenoses. Rapamycin's limited shelf life on or in an implant is to be mentioned as an additional advantage in comparison to paclitaxel because necessarily the active agent has to be effective in the first decisive weeks after stent implantation. Consequently, the endothelial cell layer which is important for the completion of a healthy healing process can completely grow over the stent and integrate it into the vessel wall.

The same mechanism of action can be found for the known derivatives of rapamycin (biolimus, everolimus, zotarolimus) as the modification is on the molecule's functional groups which are irrelevant for the binding region of mTOR. In different clinical studies (RAVEL, SIRIUS, SIROCCO) rapamycin has shown that in comparison to the strongly hydrophobic paclitaxel despite of different physical properties it is more than suitable for combating restenosis.

A further especially preferred embodiment is a valvuloplasty catheter balloon coated first (active-agent containing layer) with a mixture of rapamycin and polyethoxylated fatty alcohols, and even more preferred with a mixture of rapamycin and polyethoxylated castor oil, and coated thereafter (top coat or second layer) with polyvinyl alcohol-polyethylene glycol graft copolymer. Preferably rapamycin and polyethoxylated castor oil are used in 1.0 weight equivalents to 0.10 to 1.2 weight equivalents. Alternatively the active agent containing layer may also comprise a further additive, especially shellac or D-α-tocopherol polyethylene glycol succinate.

Coating methods for catheter balloons are disclosed in the international patent application WO 2008/086794 A2. Any kind of common coating process can be used to apply the active agent solution with or without additives like the polyethoxylated compounds and shellac, the top coat and the base coat, respectively, onto the balloon surface such as spray coating, brush coating, dip coating, vapour deposition, pipetting, drop or thread dragging, rolling, electrospinning, plasma deposition, spattering or squirting. Dipping or plasma disposition are preferably used when the whole valvuloplasty catheter balloon surface shall be coated. Spattering, brushing and spraying may be preferably used when only a portion of the balloon surface is to be coated. Accordingly, apart from dip coating specific release devices have to be used, comprising nozzles, a plurality of nozzles, a thread, a mesh of threads, a piece of threads, a leather strip, a sponge, a ball, a syringe, a needle, a cannula or a capillary.

The content of the active agent in the active agent containing solution is between 1 µg to 1 mg of the active agent per ml solution, preferably between 10 µg to 500 µg of the active agent per 1 ml solution, more preferably between 30 µg to 300 µg of the active agent per 1 ml solution, and most preferably between 50 µg to 100 µg of the active agent per 1 ml solution.

Preferred is also a total amount of 10µg to 1000 µg of an active agent per valvuloplasty catheter balloon and most preferably 20 µg to 400 µg per valvuloplasty catheter balloon.

Generally, an amount of 0.1 µg to 150 µg of active agent, preferably of paclitaxel or rapamycin, per mm² of the surface of the valvuloplasty catheter balloon to be coated can be applied onto the surface of the valvuloplasty catheter balloon, while an amount of 0.5 µg/mm² to 6 µg/mm² of active agent, preferably of paclitaxel or rapamycin, are preferred and are sufficient in order to achieve the desired effect on restenosis prophylaxis. Preferably the amount of active agent, preferably of paclitaxel or rapamycin, per mm² balloon surface is between 1.0 µg/mm² and 15.0 µg/mm², more preferably between 1.5 µg/mm² and 10.0 µg/mm², still more preferably between 2.0 µg/mm² and 6.0 µg/mm², and most preferably between 2.5 µg/mm² and 4 µg/mm².

An amount of 0.1 µg to 150 µg of the at least one polyethoxylated emulsifier or surfactant per mm² of the surface of the valvuloplasty catheter balloon to be coated can be applied onto the surface of the valvuloplasty catheter balloon, while an amount up to 15 µg/mm² of the at least one polyethoxylated emulsifier or surfactant are sufficient in order to achieve the desired effect efficient transfer of the at least one active agent to the vessel wall tissue. Preferably the amount of the at least one emulsifier or surfactant per mm² balloon surface is between 1.0 µg/mm² and 15.0 µg/mm², more preferably between 1.5 µg/mm² and 10.0 µg/mm², still more preferably between 2.0 µg/mm² and 5.0 µg/mm², and most preferably between 2.5 µg/mm² and 3.5 µg/mm².

An amount of 0.1 µg to 150 µg of polyethylene glycol per mm² of the surface of the valvuloplasty catheter balloon to be coated can be applied onto the surface of the valvuloplasty catheter balloon, while an amount up to 15 µg/mm² polyethylene glycol are sufficient in order to achieve the desired effect efficient transfer of the at least one active agent to the vessel wall tissue. Preferably the amount of polyethylene glycol per mm² balloon surface is between 1.0 µg/mm² and 15.0 µg/mm², more preferably between 1.5 µg/mm² and 10.0 µg/mm², still more preferably between 2.0 µg/mm² and 5.0 µg/mm², and most preferably between 2.5 µg/mm² and 3.5 µg/mm².

An amount of 0.1 µg to 150 µg of D-α-tocopherol polyethylene glycol succinate per mm² of the surface of the valvuloplasty catheter balloon to be coated can be applied onto the surface of the valvuloplasty catheter balloon, while an amount up to 15 µg/mm² of D-α-tocopherol polyethylene glycol succinate are sufficient in order to achieve the desired effect efficient transfer of the at least one active agent to the vessel wall tissue. Preferably the amount of D-α-tocopherol polyethylene glycol succinate per mm² balloon surface is between 1.0 µg/mm² and 15.0 µg/mm², more preferably between 1.5 µg/mm² and 10.0 µg/mm², still more preferably between 2.0 µg/mm² and 5.0 µg/mm², and most preferably between 2.5 µg/mm² and 3.5 µg/mm².

Preferred is a valvuloplasty catheter balloon having an active agent containing layer with a proportion of the active agent and the at least one polyethoxylated emulsifier or surfactant or D-α-tocopherol polyethylene glycol succinate from 90% per weight of active agent to 10% per weight of D-α-tocopherol polyethylene glycol succinate or the at least one polyethoxylated emulsifier or surfactant to 10% per weight of active agent to 90% per weight of D-α-tocopherol polyethylene glycol succinate or the at least one polyethoxylated emulsifier or surfactant. Especially preferred is a valvuloplasty catheter balloon having an active agent containing layer with a proportion of the active agent and D-α-tocopherol polyethylene glycol succinate or the at least one polyethoxylated emulsifier or surfactant from 65% per weight of active agent to 35 % per weight of D-α-tocopherol polyethylene glycol succinate or the at least one polyethoxylated emulsifier or surfactant to 35% per weight of active agent to 65% per weight of D-α-tocopherol polyethylene glycol succinate or the at least one polyethoxylated emulsifier or surfactant. Even more preferred is a valvuloplasty catheter balloon having an active agent containing layer with a proportion of the active agent and D-α-tocopherol polyethylene glycol succinate or the at least one polyethoxylated emulsifier or surfactant from 55% per weight of active agent to 45% per weight of D-α-tocopherol polyethylene glycol succinate or the at least one polyethoxylated emulsifier or surfactant to 45% per weight of active agent to 55% per weight of D-α-tocopherol polyethylene glycol succinate or the at least one polyethoxylated emulsifier or surfactant.

The further excipients or carrier substances, like shellac and polyethylene glycol, may be added in a weight ratio of up to 50% per weight relative to the at least one polyethoxylated emulsifier or surfactant used, preferably up to 40% per weight, more preferably up to 30% per weight, more preferably up to 20% per weight and especially preferably up to 10% per weight relative to the used polyethoxylated emulsifier or surfactant or D-α-tocopherol polyethylene glycol succinate.

Also preferred is a valvuloplasty catheter balloon with a coating whose molar ratio of active agent to D-α-tocopherol polyethylene glycol succinate or the at least one polyethoxylated emulsifier or surfactant and a possible further additive, like shellac and polyethylene glycol, from 90% active agent to 10% matrix substances (polyethoxylated emulsifier or surfactant and shellac) to 10% of active agent to 90% of matrix substances. Further preferred are mixtures of 1:5 to 5:1 and even more preferably from 1:2 to 2:1.

The afore-mentioned %-values are especially preferred for polyethoxylated castor oil as polyethoxylated emulsifier or surfactant.

An amount of 0.1 µg to 250 µg of top coat per mm² of the surface of the valvuloplasty catheter balloon to be coated can be applied onto the active agent coating of the valvuloplasty catheter balloon, while an amount up to 20 µg/mm² of the compound forming the top coat are sufficient in order to achieve the desired efficient transfer of the at least one active agent to the vessel wall tissue. Preferably the amount of the compound forming the top coat per mm² balloon surface is between 1.0 µg/mm² and 15.0 µg/mm², more preferably between 1.5 µg/mm² and 10.0 µg/mm², still more preferably between 2.0 µg/mm² and 5.0 µg/mm², and most preferably between 2.5 µg/mm² and 3.5 µg/mm².

An amount of 0.1 µg to 50 µg of a further additive per mm² of the surface of the valvuloplasty catheter balloon to be coated can be applied onto the surface of the valvuloplasty catheter balloon. Preferably the amount of the at least one further additive, like shellac, per mm² balloon surface is between 0.2 µg/mm² and 10.0 µg/mm², more preferably between 0.5 µg/mm² and 8.0 µg/mm², still more preferably between 1.0 µg/mm² and 5.0 µg/mm², and most preferably between 1.5 µg/mm² and 3.5 µg/mm².

Furthermore, the valvuloplasty catheter balloon can be coated in its expanded (inflated) or deflated state. According to the invention, the valvuloplasty catheter balloon does not have to be completely coated. Partial coating of the valvuloplasty catheter balloon or partial loading of certain texture elements onto the surface of the valvuloplasty catheter balloon may be sufficient.

Briefly, one suitable coating method using a coating device comprises the following steps:
A) providing an uncoated valvuloplasty catheter balloon;
B) placing the balloon into a horizontal position or inclined up to a suitable degree;
C) providing a solution of an active agent;
D) providing a solution for a top coat;
E) setting the coating device in position to transfer the solution for the active agent solution and the solution for the top coat onto the surface of the valvuloplasty catheter balloon
F) applying the respective solution
G) drying the coated valvuloplasty catheter balloon.

In step F) different solutions may be applied sequentially, wherein the solution for the top coat is always applied in a final step only followed by a final drying step.
According to the invention the solution of an active agent used in the above described coating methods may also contain D-α-tocopherol polyethylene glycol succinate or at least one polyethoxylated surfactant or at least one polyethoxylated emulsifier and/or further additives like shellac. Alternatively another solution may be provided comprising D-α-tocopherol polyethylene glycol succinate or the at least one polyethoxylated surfactant or at least one polyethoxylated and optionally shellac. Said solution should be applied to the balloon before the solution containing the active agent is applied.

Hence, according to the invention the coating method can optionally comprises further steps:
C1) providing a solution of D-α-tocopherol polyethylene glycol succinate or at least one polyethoxylated surfactant or at least one polyethoxylated emulsifier and optionally shellac or polyethylene glycol,
and
E1) applying the solution of D-α-tocopherol polyethylene glycol succinate or the at least one polyethoxylated surfactant or at least one polyethoxylated emulsifier to the valvuloplasty catheter balloon.

Step C1) is carried out following step C) and step E1) following step E). For coating by dipping, the valvuloplasty catheter balloon is dipped into a container containing the solution for the base coat, the active agent solution or solution for the top coat. Optionally drying steps can follow the application of each single solution.

Another coating method using a coating device comprises the following steps:
A') providing an uncoated valvuloplasty catheter balloon;
B') placing the balloon into a horizontal position or inclined up to a suitable degree;
C') providing a solution for a base coat;
D') providing a solution of an active agent;
E') providing a solution for a top coat;
F') setting the coating device in position to transfer the solution for the base coat, the active agent solution and the solution for the top coat onto the surface of the valvuloplasty catheter balloon
G') applying the respective solution
H') drying the coated valvuloplasty catheter balloon.

According to the invention the coating method can optionally comprises further steps:
C'1) providing a solution of D-α-tocopherol polyethylene glycol succinate or at least one polyethoxylated surfactant or at least one polyethoxylated emulsifier and optionally shellac or polyethylene glycol,
and
F'1) applying the solution of D-α-tocopherol polyethylene glycol succinate or the at least one polyethoxylated surfactant or at least one polyethoxylated emulsifier to the valvuloplasty catheter balloon.

Step C'1) is carried out following step C') and step F'1) following step F) in a way that D-α-tocopherol polyethylene glycol succinate or the at least one polyethoxylated surfactant or at least one polyethoxylated emulsifier is integrated into the coating of the valvuloplasty catheter balloon above the base layer. Alternatively, D-α-tocopherol polyethylene glycol succinate or the at least one polyethoxylated surfactant or at least one polyethoxylated emulsifier may be mixed with the active agent solution. The topcoat is always free of active agents and D-α-tocopherol polyethylene glycol succinate and the at least one polyethoxylated surfactant or at least one polyethoxylated emulsifier. This means that at least two different coating solutions have to be prepared: one with an active agent and one without for the topcoat or top layer.

For coating by dipping, the valvuloplasty catheter balloon is dipped into a container containing the solution for the base coat, the active agent solution or solution for the top coat. Optionally drying steps can follow the application of each single solution.

It is preferred that step F' is carried out in a way that the solution of the active agent penetrates the base coat.

The drying steps G) and H') can be performed at room temperature or at elevated temperatures up to 50°C and at atmospheric pressure or under reduced pressure to high vacuum. As mentioned above, the drying steps G) and H') may also be performed after application of each layer, which means a drying step is also possible subsequently after the solution of the active agent has been applied. Thereby the first drying steps are preferably conducted at room temperature and atmospheric pressure, while preferably after the last coating step of the method the drying step is more intensive, i.e. longer or with vacuum or with elevated temperature.

According to the invention the coating methods optionally comprise further step H or I') respectively:
H) or I') Sterilization of the coated valvuloplasty catheter balloons.

The sterilization is preferably performed with ethylene oxide.

Valvuloplasty catheter balloons which are coated according to the invention are preferably suited for the treatment and prophylaxis of restenosis at a heart valve, i.e. a reoccurring constriction at a treated valve.

The valvuloplasty catheter balloons coated according to the invention are preferably one component of a balloon catheter. Therefore one preferred embodiment is a balloon catheter comprising a valvuloplasty catheter balloon with a coating comprising at least one active agent and a top coat consisting of a polyvinyl alcohol - polyethylene glycol graft copolymer. Thus the present invention also relates to balloon catheter with a valvuloplasty catheter balloon coated according to the present invention. The balloon catheters according to the invention are suitable to prevent or to reduce restenosis within the area of a heart valve.

The following figures and examples illustrate potential embodiments of the invention without limiting the scope of the invention to said precise examples.

### Description of the Figures

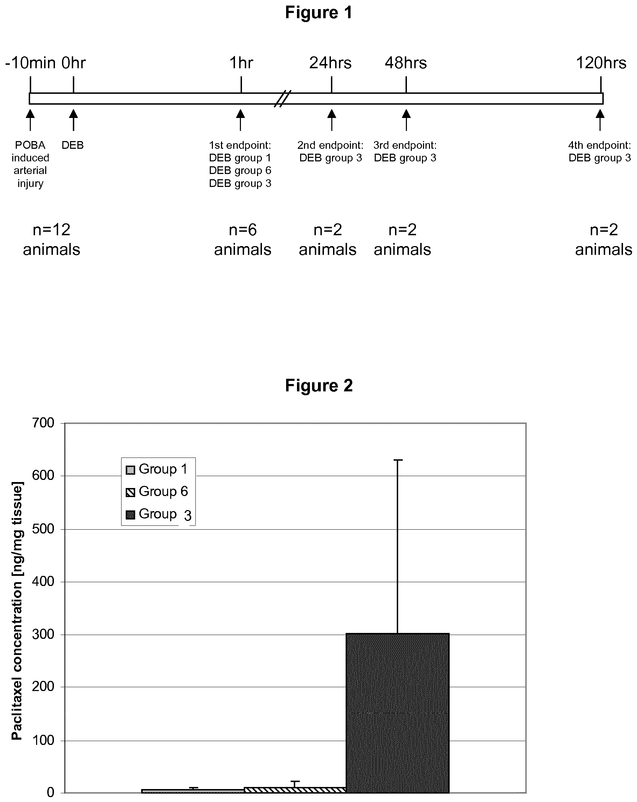

- Figure 1:: Study design and experimental procedure of example 7.
- Figure 2:: Mean tissue paclitaxel concentrations of the 3 test DEBs (example 7) one hour after deployment. The mean active agent concentrations were assessed with n= 4.

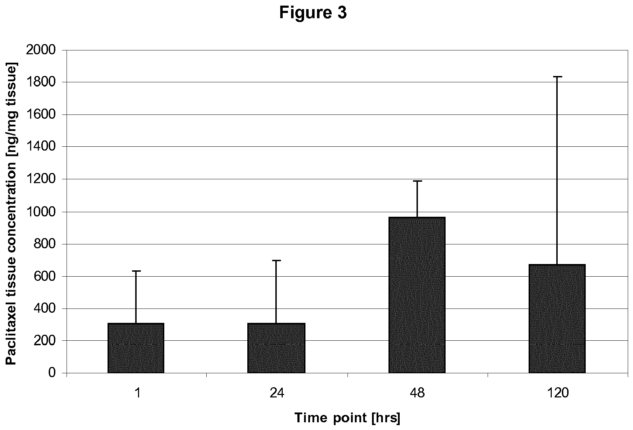

- Figure 3:: Time dependent mean tissue active agent concentrations of the Group 3 (example 7). The data are derived with n= 4 (1, 24 and 48 hours) and n=3 (120 hours).

### Examples

### Example 1

A valvuloplasty catheter balloon is provided which is coated with a solution of polyethoxylated castor oil and paclitaxel in chloroform via the drop drag method so that the final concentration of the active agent is 4.0 µg/mm² and of the emulsifier 3.0 µg/mm² of the balloon surface. The valvuloplasty catheter balloon is left to dry at room temperature for 1 h. Then a solution of top coat of a polyvinyl alcohol-polyethylene glycol graft copolymer consisting of 75% polyvinyl alcohol units and 25% polyethylene glycol units is applied onto the layer of paclitaxel. The top coating is dried at a temperature of 50°C at low vacuum.

### Example 2

A valvuloplasty catheter balloon is coated via pipetting. At first a base coat layer of polyvinyl alcohol-polyethylene glycol graft copolymer which is solved in ethanol is applied, followed by an immediate coating with a solution of paclitaxel in ethanol and polyethoxylated fatty alcohols and polyethoxylated castor oil, so that the concentration of paclitaxel is 4.0 µg/mm² and of the Polyethoxylated fatty alcohols and polyethoxylated castor oil is 3.0 µg/mm² of the balloon surface. The valvuloplasty catheter balloon is left to dry at room temperature for 1 h. Then a solution of top coat of a polyvinyl alcohol-polyethylene glycol graft copolymer consisting of 75% polyvinyl alcohol units and 25% polyethylene glycol units is applied onto the layer of paclitaxel. The top coating is dried at a temperature of 50°C at low vacuum.

### Example 3

A valvuloplasty catheter balloon is coated in a middle segment only, which consists of the middle balloon segment of a balloon for mitral valvuloplasty. The coating consists of paclitaxel and polyethoxylated fatty alcohols and polyethoxylated castor oil and is applied via the capillary method. Therefore paclitaxel and polyethoxylated fatty alcohols and polyethoxylated castor oil are solved in ethanol. The concentration of paclitaxel is 2 µg per mm² and of polyethoxylated fatty alcohols and polyethoxylated castor oil the amount is 3.0 µg/mm^{2.} Additionally a top coat of a polyvinyl alcohol-polyethylene glycol graft copolymer consisting of 75% polyvinyl alcohol units and 25% polyethylene glycol applied onto the layer of paclitaxel.

### Example 4

A valvuloplasty catheter balloon is preferably coated in a first step with a polyvinyl alcohol, and subsequently coated preferably by the squirting method with a viscous mixture of polyethylene, polyethoxylated fatty alcohols and polyethoxylated castor oil and rapamycin. Then a solution of top coat of a polyvinyl alcohol-polyethylene glycol graft copolymer consisting of 75% polyvinyl alcohol units and 25% polyethylene glycol units. The coating is dried at a temperature of 50°C at low vacuum.

### Example 5

A valvuloplasty catheter balloon is provided which is coated with a solution of D-α-tocopherol polyethylene glycol succinate and paclitaxel in ethanol via spraying so that the final concentration of paclitaxel is 3.5 µg/mm² and of the emulsifier 2.0 µg/mm² of the balloon surface. The valvuloplasty catheter balloon is left to dry at room temperature for 2 h. Then a solution of top coat of a polyvinyl alcohol-polyethylene glycol graft copolymer consisting of 75% polyvinyl alcohol units and 25% polyethylene glycol units is applied onto the layer of paclitaxel. The top coating is dried at a temperature of 50°C at low vacuum over night.

### Example 6

A valvuloplasty catheter balloon is provided which is coated with a solution of polyethoxylated castor oil, D-α-tocopherol polyethylene glycol succinate and paclitaxel in ethanol via dipping so that the final concentration of the active agent is 2.5 µg/mm² and of the amount of the mixture of emulsifier is 5.0 µg/mm² of the balloon surface. The valvuloplasty catheter balloon is left to dry at room temperature for 1 h. Then a solution of top coat of a polyvinyl alcohol-polyethylene glycol graft copolymer consisting of 75% polyvinyl alcohol units and 25% polyethylene glycol units is applied onto the layer of paclitaxel. The top coating is dried at room temperature for 24 h.

### Example 7: Proof-of-principle study for the effective active agent transfer of 6 different paclitaxel-eluting balloons (referred to as DEB) in a healthy rabbit model

This study was carried out at the 'Deutsches Herzzentrum München' - clinic at the Technical University Munich. The appropriate approval of regional Bioethical Committee was obtained. HPLC-MS-based analysis for active agent content in tissue was conducted at ic42 Laboratory, University of Colorado, USA.

Six different catheter balloons with the following coatings and carrier matrices were evaluated:
Device 1: paclitaxel-eluting balloon "Group 1", 3.0x20mm, 3.0 µg/mm² Paclitaxel, 0.5 µg/mm² Shellac, 2.5 µg/mm² Cremophor^{®} ELP (polyethoxylated castor oil) together in one layer
Device 2: paclitaxel-eluting balloon "Group 2", 3.0x20mm, 3.0 µg/mm² Paclitaxel, 3.0 µg/mm² Cremophor^{®} ELP (polyethoxylated castor oil) together in one layer and 3.0 µg/mm² PVA-PEG as a top-coat
Device 3: paclitaxel-eluting balloon "Group 3 (referred to as DEB RESTORE)", 3.0x20mm, 3.0 µg/mm² Paclitaxel, 0.5 µg/mm² Shellac, 2.5 µg/mm² Cremophor^{®} ELP (polyethoxylated castor oil) and 3.0 µg/mm² PVA-PEG as a top-coat
Device 4: paclitaxel-eluting balloon "Group 4", 3.0x20mm, 3.0 µg/mm² Paclitaxel, 3.0 µg/mm² Cremophor^{®} ELP (polyethoxylated castor oil) and 3.0 µg/mm² PEG (average molecular weight of PEG within the range of 11,000 to 12,000 Daltons) as a top-coat
Device 5: paclitaxel-eluting balloon "Group 5", 3.0x20mm, 3.0 µg/mm² Paclitaxel, and 3.0 µg/mm² PVA as a top-coat (average molecular weight of PVA within the range of 30,000 Daltons to 35,000 Daltons)
Device 6: paclitaxel-eluting balloon "Group 6", 3.0x20mm, 3.0 µg/mm² Paclitaxel together with 3 µg/mm² Cremophor^{®} ELP (polyethoxylated castor oil)

Analysis time points of arterial tissue were scheduled at 1 hour, 24 hours, 48 hours and 120 hours.

### Study design:

A total of 47 DEBs were deployed in 24 healthy New Zealand White rabbits (please refer to table 3). For this purpose animals were anaesthetized with propofol and intra surgery analgesia was secured by repetitive boli of fentanyl. Animals were intubated, mechanically ventilated and at all times controlled for vital signs (pulse-oximetry and capnography). Anticoagulation was achieved by administration of 500 IU heparin and 40 mg aspirin i.v. Arterial access was conducted by cut down of the common carotid artery. A swan ganz catheter was advanced over the aortic arch under fluoroscopic guidance just before the bifurcation of the common iliac arteries of the abdominal aorta and an initial angiogram was performed. A guide wire was then placed in the external iliac artery. Wire guided balloon injury (POBA) with single balloon inflation [3,0x10mm size balloon (Elect^{®}, Biotronik SE & Co. KG) at nominal pressure (7atm) held for 30 seconds] within the middle portion of the external iliac artery was performed to induce arterial injury and facilitate active agent uptake into the vascular wall of healthy arteries. Afterwards the DEB (3,0x20mm) was deployed covering the whole length of the induced lesion. The DEBs were inflated at nominal pressure (6 atm) for 60 seconds. Per time point and group, 4 DEBs were bilaterally deployed in the iliac arteries of 2 rabbits utilizing the technique explained above. Five minutes after the procedure, a final angiogram was conducted. To determine DEBs with effective active agent delivery capability to the arterial tissue, all DEB groups were assessed at 1 hour following deployment. The DEB showing the most promising tissue active agent content (Group 3, DEB-RESTORE) was then further analyzed at 24 hours, 48 hours and 120 hours following DEB expansion. Animals with a study in-life phase (time point 48 and 120 hours) received oral anticoagulation once daily the day after surgery with 40mg aspirin until study termination.

**Table 1: Scheme of Implantation**

| Animal Number | Animal ID | Time Point | Vessel | |
|---|---|---|---|---|
| | | | Left iliac artery | Right iliac artery |
| 1 | 1_12 | 1 hour | Group 1 #2 | Group 1 #1 |
| 2 | 2_12 | 1 hour | Group 1 #3 | Group 1 #4 |
| 3 | 3_12 | 1 hour | Group 3 #1 | Group 3 #2 |
| 4 | 4_12 | 1 hour | Group 3 #4 | Group 3 #3 |
| 5 | 5_12 | 1 hour | Group 6 #2 | Group 6 #1 |
| 6 | 6_12 | 1 hour | Group 6 #3 | Group 6 #4 |
| 13 | 13_12 | 1 hour | Group 2 #2 | Group 2 #1 |
| 14 | 14_12 | 1 hour | Group 2 #4 | Group 2 #3 |
| 15 | 15_12 | 1 hour | Group 4 #1 | Group 4 #2 |
| 16 | 16_12 | 1 hour | Group 4 #3 | Group 4 #4 |
| 17 | 17_12 | 1 hour | Group 5 #2 | Group 5 #1 |
| 18 | 18_12 | 1 hour | Group 5 #3 | Group 5 #4 |
| 7 | 7_12 A | 24 hours | Group 3 #6 | Group 3 #5 |
| 8 | 8_12 A | 24 hours | Group 3 #8 | Group 3 #7 |
| 9 | 9_12 | 48 hours | Group 3 #10 | Group 3 #9 |
| 10 | 10_12 | 48 hours | Group 3 #15 | Group 3 #14 |
| 11 | 11_12 | 120 hours | Group 3 #11 | Group 3 #12 |
| 12 | 12_12 | 120 hours | Group 3 #13 | none |

24 animals were assigned to the study. The first 18 animals were analyzed for arterial tissue active agent content at 1 hour following deployment of all test DEBs. The remaining 6 animals were assigned to the Group 3 and analyzed after 24, 48 and 120 hours. Prior to DEB deployment the external iliac arteries were injured by inflating an angioplasty balloon catheter (Elect^{©}, Biotronik SE & Co. KG, 3.0x10mm, 7 atm inflation pressure) within the middle portions of the arteries for 30 seconds.

For study termination animals were anaesthetized at the respective time points and shortly after euthanized with pentobarbital overdose i.v. Animals of the 1 hour group remained under anaesthesia until study termination and tissue harvest. Following euthanasia the abdomen was cut open and the abdominal aorta and caudal vena cava were exposed and accessed with arterial sheaths. Consecutively the vessels were flushed with 500 ml heparinized Ringers solution via the arterial sheath until blood clearance. Treated external iliac arteries were then carefully dissected, explanted and snap frozen in liquid nitrogen. Eighteen animals were euthanized 1 hour following DEB deployment including all designated treatment groups (n=4 arteries per group), while the remaining six animals of the Group 3 were euthanized at 24 hours, 48 hours and 5 days following balloon expansion (n=4 arteries for the 24 and 48 hour time point; n=3 arteries for the 120 hour time point). Treated iliac arteries were stored at -70°C until shipment on dry ice to the analytic laboratory. Explanted treated vessels were weighed, homogenized and the undiluted homogenate was measured for paclitaxel content. At all time points, the batch samples (1 st batch = 1 hour samples of the test DEBs; 2nd batch = 24, 48 and 120 hour samples of the Group 3, DEB-RESTORE) were clearly identified and were processed on the same day using the same extraction method. Samples showing over detection range paclitaxel content were diluted 1:100 and 1:500 and were repeatedly measured.

### Results:

All animals survived the procedure without signs of toxicity. There were no adverse effects noted after DEB deployment and the post DEB expansion angiography showed patent vessels and no sign of vessel wall dissection. Macroscopically, at the time of vessel explantation, there were also no signs of vessel trauma or dissection. Of note was that the vessels of the animal 8_12 A (24 hour Group 3) and of the animals 9_12 and 10_12 (both animals of the 48 hour Group 3) showed 1-2mm long white depositions located at the ventral site of the arteries. This phenomenon was not observed in the 1 hour and 120 hour groups. Animals with an in-life phase of the study (24, 48 and 120 hour Group 3) showed no changes in blood perfusion of the treated legs as assessed clinically by daily palpation of the femoral artery pulse and checking the toes for signs of hypoxia.

HPLC-based analysis of the tissue active agent concentrations revealed that the DEB group 1 exhibited an active agent concentration of 5.19 ± 3.95 ng active agent/mg tissue (n=4 arteries) in the arterial wall 1 hour after DEB deployment. The tissue concentrations ranged from 1 to 10 ng/mg.
The mean tissue active agent concentration analyzed for Group 6 in the arterial wall 1 hour after DEB deployment was 10.72 ± 11.24 ng active agent/mg tissue (n=4 arteries). The tissue concentrations ranged from 4 to 27 ng/mg.

One hour after balloon expansion the Group 3 showed a high amount of active agent within the arterial tissue as represented by a mean paclitaxel concentration of 303.29 ± 326.98 ng active agent/mg tissue (n=4 arteries). Notably, there was a high variability in the arterial wall active agent up-take ranging from 2 to >700 ng/mg tissue. Analysis of arterial active agent concentration at later time points revealed that high concentrations of active agent were still encountered for up to 120 hours (resembling the latest analyzed time point).

24 hours after Group 3 deployment the mean tissue concentration of paclitaxel was 302.65 ± 391.71 ng active agent/mg tissue (n=4 arteries). At 48 hours, a rise in tissue active agent concentration up to a mean value of 961.94 ± 226.54 ng active agent/mg tissue was denoted within the 4 analyzed vessels. At the latest analyzed time point of 120 hours, there was a slight decrease in mean active agent concentration (674.26 ± 1158.78 ng active agent/mg tissue). Yet there was a strong variability of tissue active agent concentrations between the treated vessels ranging from 4 to >2000 ng/mg (n=3 arteries).

**Table 2: Mean active agent tissue concentrations (ng active agent/mg tissue)**

| Time point | Device Grouping | | |
|---|---|---|---|
| | Group 1 | Group 6 | Group 3 |
| 1 hour (n=4) | 5.19 ± 3.95 | 10.72 ± 11.24 | 303.29 ± 326.98 |
| 24 hours (n=4) | Not performed | Not performed | 302.65 ± 391.71 |
| 48 hours (n=4) | Not performed | Not performed | 961.94 ± 226.54 |
| 120 hours (n=4) | Not performed | Not performed | 674.26 ± 1158.978 |

The observed divergences in active agent up-take were within the expected range when utilizing healthy animal models, as active agent up-take in healthy vessels is primarily dependent on the degree of tissue injury prior to DEB deployment. In one case (Group 3, Balloon number 5, 24 hour time point) the denoted low active agent up-take can be explained by loss of the paclitaxel coating during extraction of the protective sheath.

Among the test devices the Group 3 achieved significant paclitaxel concentrations that were previously shown to be effective in neointimal growth reduction among contemporary drug eluting balloon devices (Unverdorben et al, Circ 2009; Joner et al, Thromb Haemost 2011).
As a consequence the Group 3 was further analyzed for its pharmacokinetic profile up to 120 hours (5 days) following deployment. The application of this device appears to be safe as there was no evidence for vessel wall dissections or aneurysms at follow-up for up to 5 days and animals also showed no signs of malperfusion of the treated legs suggesting absence of embolization. According to the findings of the current study, there was a rapid active agent up-take of paclitaxel within the arterial tissue within the first 24 hours. Of note was that tissue concentrations further increased within 24 and 48 hours following DEB-RESTORE deployment which provides evidence for the delayed active agent up-take capacity of the Group 3 when deployed in healthy arteries. This is recognized as a favorable effect of contemporary drug eluting balloons, as the prolonged bioavailability within the vessel wall is a hallmark of enhanced antiproliferative potential.

### Conclusion:

This proof-of-principle study showed that among the devices tested, the balloon according to the invention comprising a top coat (Group 3) allows for the accumulation of therapeutic active agent concentrations in the arterial wall for at least 5 days, with peak tissue accumulation after 48 hours post deployment.

## Claims

1. A valvuloplasty catheter balloon with a coating comprising at least one active agent and a top coat consisting of a polyvinyl alcohol - polyethylene glycol graft copolymer.

2. Valvuloplasty catheter balloon according to claim 1, wherein the polyvinyl alcohol - polyethylene glycol graft copolymer consists of 75% polyvinyl alcohol units and 25% polyethylene glycol units.

3. Valvuloplasty catheter balloon according to claim 1 or 2 wherein the coating further comprises at least one polyethoxylated surfactant, at least one polyethoxylated emulsifier, D-α-Tocopherol polyethylene glycol succinate or polyethylene glycol.

4. Valvuloplasty catheter balloon according to claim 3, wherein the coating further comprises D-α-Tocopherol polyethylene glycol succinate.

5. Valvuloplasty catheter balloon according to claim 3, wherein the at least one polyethoxylated surfactant or at least one polyethoxylated emulsifier is selected from the group consisting of:
polyethoxylated alcohols, polyethoxylated oils, polyethoxylated castor oil, polyethoxylated glycerol, polyethoxylated fatty acid esters, polyethoxylated phenols, polyethoxylated amines, and polyethoxylated fatty alcohols.

6. Valvuloplasty catheter balloon according to claim 3 or 5 wherein the at least one polyethoxylated surfactant or at least one polyethoxylated emulsifier is a polyethoxylated castor oil.

7. Valvuloplasty catheter balloon according to any of the claims 3, 5 or 6, wherein the at least one polyethoxylated surfactant or at least one polyethoxylated emulsifier is prepared by reacting castor oil with ethylene oxide in a molar ratio of 1:35.

8. Valvuloplasty catheter balloon according to claim 6 or 7, wherein the at least one polyethoxylated surfactant or at least one polyethoxylated emulsifier is further purified to remove potassium ions and free fatty acids.

9. Valvuloplasty catheter balloon according to any of the claims 1 - 8, wherein the at least one active agent is an antiproliferative, immunosuppressive, anti-angiogenic, anti-inflammatory, anti-restenotic, and/or anti-thrombotic agent.

10. Valvuloplasty catheter balloon according to claim 9, wherein the at least one antiproliferative, immunosuppressive, anti-angiogenic, anti-inflammatory, anti-restenotic, and/or anti-thrombotic agent is selected from the group consisting of: abciximab, acemetacin, acetylvismione B, aclarubicin, ademetionine, adriamycin, aescin, afromosone, akagerine, aldesleukin, amidorone, aminoglutethimide, amsacrine, anakinra, anastrozole, anemonin, anopterine, antimycotics antithrombotics, apocymarin, argatroban, aristolactam-All, aristolochic acid, ascomycin, asparaginase, aspirin, atorvastatin, auranofin, azathioprine, azithromycin, baccatin, bafilomycin, basiliximab, bendamustine, benzocaine, berberine, betulin, betulinic acid, bilobol, bisparthenolidine, bleomycin, combrestatin, Boswellic acids, bruceanol A, B and C, bryophyllin A, busulfan, antithrombin, bivalirudin, cadherins, camptothecin, capecitabine, o-carbamoyl-phenoxyacetic acid, carboplatin, carmustine, celecoxib, cepharanthin, cerivastatin, CETP inhibitors, chlorambucil, chloroquine phosphate, cicutoxin, ciprofloxacin, cisplatin, cladribine, clarithromycin, colchicine, concanamycin, coumadin, C-type natriuretic peptide (CNP), cudraisoflavone A, curcumin, cyclophosphamide, ciclosporin A, cytarabine, dacarbazine, daclizumab, dactinomycin, dapsone, daunorubicin, diclofenac, 1,11-dimethoxycanthin-6-one, docetaxel, doxorubicin, daunamycin, epirubicin, erythromycin, estramustine, etoposide, filgrastim, fluroblastin, fluvastatin, fludarabine, fludarabine-5'-dihydrogen phosphate, fluorouracil, folimycin, fosfestrol, gemcitabine, ghalakinoside, ginkgol, ginkgolic acid, glycoside 1a, 4-hydroxyoxycyclo phosphamide, idarubicin, ifosfamide, josamycin, lapachol, lomustine, lovastatin, melphalan, midecamycin, mitoxantrone, nimustine, pitavastatin, pravastatin, procarbazine, mitomycin, methotrexate, mercaptopurine, thioguanine, oxaliplatin, irinotecan, topotecan, hydroxycarbamide, miltefosine, pentostatin, pegaspargase, exemestane, letrozole, formestane, mycophenolate mofetil, β-lapachone, podophyllotoxin, podophyllic acid-2-ethyl hydrazide, molgramostim (rhuGM-CSF), peginterferon α-2b, lenograstim (r-HuG-CSF), macrogol, selectin (cytokine antagonist), cytokinin inhibitors, COX-2 inhibitor, angiopeptin, monoclonal antibodies inhibiting muscle cell proliferation, bFGF antagonists, probucol, prostaglandins, 1-hydroxy-11-methoxycanthin-6-one, scopoletin, NO donors, pentaerythrityl tetranitrate and sydnoimines, tamoxifen, staurosporine, β-estradiol, α-estradiol, estriol, estrone, ethinyl estradiol, medroxyprogesterone, estradiol cypionates, estradiol benzoates, tranilast, kamebakaurin and other terpenoids used in cancer therapy, verapamil, tyrosine kinase inhibitors (tyrphostins), paclitaxel, 6-α-hydroxy-paclitaxel, taxoteres, mofebutazone, lonazolac, lidocaine, ketoprofen, mefenamic acid, piroxicam, meloxicam, penicillamine, hydroxychloroquine, sodium aurothiomalate, oxaceprol, β-sitosterol, myrtecaine, polidocanol, nonivamide, levomenthol, ellipticine, D-24851 (Calbiochem), colcemid, cytochalasin A-E, indanocine, nocodazole, bacitracin, vitronectin receptor antagonists, azelastine, guanidyl cyclase stimulator, tissue inhibitor of metal proteinase-1 and -2, free nucleic acids, nucleic acids incorporated into virus transmitters, DNA and RNA fragments, plasminogen activator inhibitor 1, plasminogen activator inhibitor 2, antisense oligonucleotides, VEGF inhibitors, IGF-1, active agents from the group of antibiotics, cefadroxil, cefazolin, cefaclor, cefoxitin, tobramycin, gentamicin, penicillins, dicloxacillin, oxacillin, sulfonamides, metronidazole, enoxaparin, heparin, hirudin, PPACK, protamine, prourokinase, streptokinase, warfarin, urokinase, vasodilators, dipyramidole, trapidil, nitroprussides, PDGF antagonists, triazolopyrimidine, seramin, ACE inhibitors, captopril, cilazapril, lisinopril, enalapril, losartan, thioprotease inhibitors, prostacyclin, vapiprost, interferon α, β and γ, histamine antagonists, serotonin blockers, apoptosis inhibitors, apoptosis regulators, halofuginone, nifedipine, tocopherol, tranilast, molsidomine, tea polyphenols, epicatechin gallate, epigallocatechin gallate, leflunomide, etanercept, sulfasalazine, tetracycline, triamcinolone, mutamycin, procainimide, retinoic acid, quinidine, disopyrimide, flecainide, propafenone, sotalol, natural and synthetically obtained steroids such as bryophyllin A, inotodiol, maquiroside A, ghalakinoside, mansonine, strebloside, hydrocortisone, betamethasone, dexamethasone, non-steroidal substances (NSAIDS), fenoprofen, ibuprofen, indomethacin, naproxen, phenylbutazone, antiviral agents, acyclovir, ganciclovir zidovudine, clotrimazole, flucytosine, griseofulvin, ketoconazole, miconazole, nystatin, terbinafine, antiprotozoal agents, chloroquine, mefloquine, quinine, natural terpenoids, hippocaesculin, barringtogenol-C21-angelate, 14-dehydroagrostistachin, agroskerin, agrostistachin, 17-hydroxyagrostistachin, ovatodiolids, 4,7-oxycycloanisomelic acid baccharinoids B1, B2, B3 and B7, tubeimoside, bruceantinoside C, yadanziosides N and P, isodeoxyelephantopin, tomenphantopin A and B, coronarin A,B C and D, ursolic acid, hyptatic acid A, iso-iridogermanal, maytenfoliol, effusantin A, excisanin A and B, longikaurin B, sculponeatin C, kamebaunin, leukamenin A and B, 13,18-dehydro-6-alpha-senecioyloxychaparrin, taxamairin A and B, regenilol, triptolide, cymarin, hydroxyanopterine, protoanemonin, cheliburin chloride, sinococuline A and B, dihydronitidine, nitidine chloride, 12-β-hydroxypregnadien-3,20-dione, helenalin, indicine, indicine-N-oxide, lasiocarpine, inotodiol, podophyllotoxin, justicidin A and B, larreatin, malloterin, mallotochromanol, isobutyrylmallotochromanol, marchantin A, maytansin, lycoridicin, margetine, pancratistatin, liriodenine, oxoushinsunine, periplocoside A, deoxypsorospermin, psychorubin, ricin A, sanguinarine, manwu wheat acid, methylsorbifolin, chromones of spathelia, stizophyllin, dihydrousambaraensine, hydroxyusambarine, strychnopentamine, strychnophylline, usambarine, usambarensine, liriodenine, daphnoretin, lariciresinol, methoxylariciresinol, syringaresinol, sirolimus (rapamycin), biolimus A9, pimecrolimus, everolimus, myolimus, novolimus, ridaforolimus, temsirolimus, zotarolimus, tacrolimus, fasudil, epothilones, somatostatin, roxithromycin, troleandomycin, simvastatin, rosuvastatin, vinblastine, vincristine, vindesine, teniposide, vinorelbine, trofosfamide, treosulfan, temozolomide, thiotepa, tretinoin, spiramycin, umbelliferone, desacetylvismione A, vismione A and B, zeorin.

11. Valvuloplasty catheter balloon according to claim 10, wherein the at least one active agent is selected from the group consisting of:
paclitaxel, taxanes, docetaxel, sirolimus, biolimus A9, pimecrolimus, everolimus, myolimus, novolimus, ridaforolimus, temsirolimus, zotarolimus, tacrolimus, fasudil and epothilones.

12. Valvuloplasty catheter balloon according to one of the claims 1 - 11, wherein the coating further comprises shellac.

13. Valvuloplasty catheter balloon according to any of the claims 1 - 12, wherein the coating further comprises a base coat on said catheter balloon consisting of polyvinyl alcohol-polyethylene glycol graft copolymer and/or shellac.

14. Balloon catheter comprising a valvuloplasty catheter balloon according to any of the claims 1 - 13.

15. Balloon catheter according to claim 14 suitable to prevent or to reduce restenosis at a heart valve.

## Patentansprüche

1. Ein Valvuloplastie-Katheterballon mit einer Beschichtung, umfassend mindestens einen Wirkstoff und eine aus einem Polyvinylalkohol-Polyethylenglycol-Pfropfcopolymer bestehende Deckschicht.

2. Valvuloplastie-Katheterballon gemäß Anspruch 1, wobei das Polyvinylalkohol-Polyethylenglycol-Pfropfcopolymer aus 75% Polyvinylalkoholeinheiten und 25% Polyethylenglycoleinheiten besteht.

3. Valvuloplastie-Katheterballon gemäß Anspruch 1 oder 2, wobei die Beschichtung weiter mindestens ein polyethoxyliertes Tensid, mindestens einen polyethoxylierten Emulgator, D-α-Tocopherolpolyethylenglycolsuccinat oder Polyethylenglycol umfasst.

4. Valvuloplastie-Katheterballon gemäß Anspruch 3, wobei die Beschichtung weiter D-α-Tocopherolpolyethylenglycolsuccinat umfasst.

5. Valvuloplastie-Katheterballon gemäß Anspruch 3, wobei das mindestens eine polyethoxylierte Tensid oder der mindestens eine polyethoxylierte Emulgator ausgewählt ist aus der Gruppe bestehend aus:
polyethoxylierte Alkohole, polyethoxylierte Öle, polyethoxyliertes Castoröl, polyethoxyliertes Glycerol, polyethoxylierte Fettsäureester, polyethoxylierte Phenole, polyethoxylierte Amine und polyethoxylierte Fettalkohole.

6. Valvuloplastie-Katheterballon gemäß Anspruch 3 oder 5, wobei das mindestens eine polyethoxylierte Tensid oder der mindestens eine polyethoxylierte Emulgator ein polyethoxyliertes Castoröl ist.

7. Valvuloplastie-Katheterballon gemäß einem der Ansprüche 3, 5 oder 6, wobei das mindestens eine polyethoxylierte Tensid oder der mindestens eine polyethoxylierte Emulgator durch Reagieren von Castoröl mit Ethylenoxid in einem molaren Verhältnis von 1:35 hergestellt wird.

8. Valvuloplastie-Katheterballon gemäß Anspruch 6 oder 7, wobei das mindestens eine polyethoxylierte Tensid oder der mindestens eine polyethoxylierte Emulgator weiter gereinigt wird, um Kaliumionen und freie Fettsäuren zu entfernen.

9. Valvuloplastie-Katheterballon gemäß einem der Ansprüche 1 - 8, wobei der mindestens eine Wirkstoff ein antiproliferatives, immunsuppressives, antiangiogenes, entzündungshemmendes, antirestenotisches und/oder antithrombotisches Mittel ist.

10. Valvuloplastie-Katheterballon gemäß Anspruch 9, wobei das mindestnes eine antiproliferative, immunsuppressive, anti-angiogene, entzündungshemmende, antirestenotische und/oder antithrombotische Mittel ausgewählt ist aus der Gruppe bestehend aus: Abciximab, Acemetacin, Acetylvismion B, Aclarubicin, Ademetionin, Adriamycin, Aescin, Afromoson, Akagerin, Aldesleukin, Amidoron, Aminoglutethimid, Amsacrin, Anakinra, Anastrozol, Anemonin, Anopterin, Antimykotika Antithrombotika, Apocymarin, Argatroban, Aristolactam-All, Aristolochsäure, Ascomycin, Asparaginase, Aspirin, Atorvastatin, Auranofin, Azathioprin, Azithromycin, Baccatin, Bafilomycin, Basiliximab, Bendamustin, Benzocain, Berberin, Betulin, Betulinsäure, Bilobol, Bisparthenolidin, Bleomycin, Bombrestatin, Boswellinsäuren, Bruceanol A, B und C, Bryophyllin A, Busulfan, Antithrombin, Bivalirudin, Cadherine, Camptothecin, Capecitabin, o-Carbamoylphenoxyessigsäure, Carboplatin, Carmustin, Celecoxib, Cepharanthin, Cerivastatin, CETP-Inhibitoren, Chlorambucil, Chlorochinphosphat, Cicutoxin, Ciprofloxacin, Cisplatin, Cladribin, Clarithromycin, Colchicin, Concanamycin, Coumadin, C-Typ-natriuretisches Peptid (CNP), Cudraisoflavon A, Curcumin, Cyclophosphamid, Ciclosporin A, Cytarabin, Dacarbazin, Daclizumab, Dactinomycin, Dapson, Daunorubicin, Diclofenac, 1,11-Dimethoxycanthin-6-on, Docetaxel, Doxorubicin, Daunamycin, Epirubicin, Erythromycin, Estramustin, Etoposid, Filgrastim, Fluroblastin, Fluvastatin, Fludarabin, Fludarabin-5'-dihydrogenphosphat, Fluorouracil, Folimycin, Fosfestrol, Gemcitabin, Ghalakinosid, Ginkgol, Ginkgolsäure, Glykosid 1a, 4Hydroxyoxycyclophosphamid, Idarubicin, Ifosfamid, Josamycin, Lapachol, Lomustin, Lovastatin, Melphalan, Midecamycin, Mitoxantron, Nimustin, Pitavastatin, Pravastatin, Procarbazin, Mitomycin, Methotrexat, Mercaptopurin, Thioguanin, Oxaliplatin, Irinotecan, Topotecan, Hydroxycarbamid, Miltefosin, Pentostatin, Pegaspargase, Exemestan, Letrozol, Formestan, Mycophenolatmofetil, β-Lapachon, Podophyllotoxin, Podophyllsäure-2-ethylhydrazid, . Molgramostim (rhuGM-CSF), Peginterferon α-2b, Lenograstim (r-HuG-CSF), Macrogol, Selectin (Cytokinantagonist), Cytokinininhibitoren, COX-2-lnhibitor, Angiopeptin, monoklonale Antikörper, die die Muskelzellproliferation hemmen, bFGF-Antagonisten, Probucol, Prostaglandine, 1-Hydroxy-11-methoxycanthin-6-on, Scopoletin, NO-Donoren, Pentaerythrityltetranitrat und Sydnoimine, Tamoxifen, Staurosporin, β-Estradiol, α-Estradiol, Estriol, Estron, Ethinylestradiol, Medroxyprogesteron, Estradiolcypionate, Estradiolbenzoate, Tranilast, Kamebakaurin und andere Terpenoide, die in der Krebstherapie eingesetzt werden, Verapamil, Tyrosinkinase-Inhibitoren (Tyrphostine), Paclitaxel, 6-α-Hydroxypaclitaxel, Taxotere, Mofebutazon, Lonazolac, Lidocain, Ketoprofen, Mefenaminsäure, Piroxicam, Meloxicam, Penicillamin, Hydroxychlorochin, Natriumaurothiomalat, Oxaceprol, β-Sitosterol, Myrtecain, Polidocanol, Nonivamid, Levomenthol, Ellipticin, D-24851 (Calbiochem), Colcemid, Cytochalasin A-E, Indanocin, Nocodazol, Bacitracin, Vitronectin-Rezeptor Antagonisten, Azelastin, Guanidylcyclase-Stimulator, Gewebsinhibitor der Metallproteinase-1 und 2, freie Nukleinsäuren, Nukleinsäuren in Virenüberträger inkorporiert, DNA- und RNA-Fragmente, Plasminogen-Aktivator lnhibitor-1, Plasminogen-Aktivator Inhibitor-2, Antisense Oligonukleotide, VEGF-Inhibitoren, IGF-1, Wirkstoffe aus der Gruppe der Antibiotika, Cefadroxil, Cefazolin, Cefaclor, Cefoxitin, Tobramycin, Gentamicin, Penicilline, Dicloxacillin, Oxacillin, Sulfonamide, Metronidazol, Enoxaparin, Heparin, Hirudin, PPACK, Protamin, Prourokinase, Streptokinase, Warfarin, Urokinase, Vasodilatoren, Dipyramidol, Trapidil, Nitroprusside, PDGF-Antagonisten, Triazolopyrimidin, Seramin, ACE-Inhibitoren, Captopril, Cilazapril, Lisinopril, Enalapril, Losartan, Thioprotease-Inhibitoren, Prostacyclin, Vapiprost, Interferon α, β und γ, Histamin-Antagonisten, Serotonin-Blocker, Apoptose-Inhibitoren, Apoptose-Regulatoren, Halofuginon, Nifedipin, Tocopherol, Tranilast, Molsidomin, Teepolyphenole, Epicatechingallat, Epigallocatechingallat, Leflunomid, Etanercept, Sulfasalazin, Tetracyclin, Triamcinolon, Mutamycin, Procainimid, Retinolsäure, Quinidin, Disopyrimid, Flecainid, Propafenon, Sotalol, natürliche und synthetisch hergestellte Steroide wie Bryophyllin A, Inotodiol, Maquirosid A, Ghalakinosid, Mansonin, Streblosid, Hydrocortison, Betamethason, Dexamethason, nichtsteroidale Substanzen (NSAIDS), Fenoprofen, Ibuprofen, Indomethacin, Naproxen, Phenylbutazon, antivirale Agentien, Acyclovir, Ganciclovir Zidovudin, Clotrimazol, Flucytosin, Griseofulvin, Ketoconazol, Miconazol, Nystatin, Terbinafin, antiprotozoale Agenzien, Chloroquin, Mefloquin, Quinin, natürliche Terpenoide, Hippocaesculin, Barringtogenol-C21-angelat, 14-Dehydroagrostistachin, Agroskerin, Agrostistachin, 17-Hydroxyagrostistachin, Ovatodiolid, 4,7 Oxycycloanisomelsäure, Baccharinoide B1, B2, B3 und B7, Tubeimosid, Bruceantinosid C, Yadanzioside N und P, Isodeoxyelephantopin, Tomenphantopin A und B, Coronarin A,B C und D, Ursolsäure, Hyptatatsäure A, Iso-Iridogermanal, Maytenfoliol, Effusantin A, Excisanin A und B, Longikaurin B, Sculponeatin C, Kamebaunin, Leukamenin A und B, 13,18-Dehydro-6-alpha-Senecioyloxychaparrin, Taxamairin A und B, Regenilol, Triptolid, Cymarin, Hydroxyanopterin, Protoanemonin, Cheliburinchlorid, Sinococulin A and B, Dihydronitidin, Nitidinchlorid, 12-β-Hydroxypregnadien-3,20-dion, Helenalin, Indicin, Indicin-N-oxid, Lasiocarpin, Inotodiol, Podophyllotoxin, Justicidin A und B, Larreatin, Malloterin, Mallotochromanol, Isobutyrylmallotochromanol, Marchantin A, Maytansin, Lycoridicin, Margetin, Pancratistatin, Liriodenin, Oxoushinsunin, Periplocosid A, Deoxypsorospermin, Psychorubin, Ricin A, Sanguinarin, Manwuweizsäure, Methylsorbifolin, Chromone der Spathelia, Stizophyllin, Dihydrousambaraensin, Hydroxyusambarin, Strychnopentamin, Strychnophyllin, Usambarin, Usambarensin, Liriodenin, Daphnoretin, Lariciresinol, Methoxylariciresinol, Syringaresinol, Sirolimus (Rapamycin), Biolimus A9, Pimecrolimus, Everolimus, Zotarolimus, Tacrolimus, Fasudil, Epothilone, Somatostatin, Roxithromycin, Troleandomycin, Simvastatin, Rosuvastatin, Vinblastin, Vincristin, Vindesin, Teniposid, Vinorelbin, Trofosfamid, Treosulfan, Temozolomid, Thiotepa, Tretinoin, Spiramycin, Umbelliferon, Desacetylvismion A, Vismion A und B, Zeorin.

11. Valvuloplastie-Katheterballon gemäß Anspruch 10, wobei der mindestens eine Wirkstoff ausgewählt ist aus der Gruppe bestehend aus:
Paclitaxel, Taxane, Docetaxel, Sirolimus, Biolimus A9, Pimecrolimus, Everolimus, Myolimus, Novolimus, Ridaforolimus, Temsirolimus, Zotarolimus, Tacrolimus, Fasudil und Epothilone

12. Valvuloplastie-Katheterballon gemäß einem der Ansprüche 1 - 11. wobei die Beschichtung weiter Schellack umfasst.

13. Valvuloplastie-Katheterballon gemäß einem der Ansprüche 1 - 12. wobei die Beschichtung weiter eine aus einem Polyvinylalkohol-Polyethylenglycol-Pfropfcopolymer und/oder Schellack bestehende Grundschicht auf besagtem Katheterballon umfasst.

14. Ballonkatheter umfassend einen Valvulopastie-Katheterballon gemäß einem der Ansrpüche 1 - 13.

15. Ballonkatheter gemäß Anspruch 14 geeignet zur Prävention oder Reduzierung von Restenose an einer Herzklappe.

## Revendications

1. Un ballonnet de cathéter pour valvuloplastie avec un revêtement comprenant au moins un agent actif et une couche supérieure constituée d'un copolymère greffé d'alcool polyvinylique - polyéthylène glycol.

2. Ballonnet de cathéter pour valvuloplastie selon la revendication 1, dans lequel le copolymère greffé d'alcool polyvinylique - polyéthylène glycol contient 75 % d'unités d'alcool polyvinylique et 25 % d'unités de polyéthylène glycol.

3. Ballonnet de cathéter pour valvuloplastie selon la revendication 1 ou 2 dans lequel le revêtement comprend en outre au moins un tensioactif polyéthoxylé, au moins un émulsifiant polyéthoxylé, le polyéthylène glycol succinate de D-α-tocophérol ou le polyéthylène glycol.

4. Ballonnet de cathéter pour valvuloplastie selon la revendication 3, dans lequel le revêtement comprend en outre le polyéthylène glycol succinate de D-α-tocophérol.

5. Ballonnet de cathéter pour valvuloplastie selon la revendication 3, dans lequel le tensioactif polyéthoxylé ou l'émulsifiant polyéthoxylé est sélectionné parmi le groupe comprenant :
les alcools polyéthoxylés, les huiles polyéthoxylées, l'huile de ricin polyéthoxylée, le glycérol polyéthoxylé, les esters d'acides gras polyéthoxylés, les phénols polyéthoxylés, les amines polyéthoxylées et les alcools gras polyéthoxylés.

6. Ballonnet de cathéter pour valvuloplastie selon la revendication 3 ou 5 dans lequel le tensioactif polyéthoxylé ou l'émulsifiant polyéthoxylé est une huile de ricin polyéthoxylée.

7. Ballonnet de cathéter pour valvuloplastie selon l'une quelconque des revendications 3, 5 ou 6, dans lequel le tensioactif polyéthoxylé ou l'émulsifiant polyéthoxylé est préparé par la réaction entre l'huile de ricin et l'oxyde d'éthylène dans un rapport molaire de 1:35.

8. Ballonnet de cathéter pour valvuloplastie selon la revendication 6 ou 7, dans lequel le tensioactif polyéthoxylé ou l'émulsifiant polyéthoxylé est ensuite purifié pour éliminer les ions de potassium et les acides gras libres.

9. Ballonnet de cathéter pour valvuloplastie selon l'une quelconque des revendications de 1 à 8, dans lequel l'agent actif est un agent antiprolifératif, immunosuppresseur, anti-angiogénique, anti-inflammatoire, anti-resténose et / ou anti-thrombotique.

10. Ballonnet de cathéter pour valvuloplastie selon la revendication 9, dans lequel l'agent antiprolifératif, immunosuppresseur, anti-angiogénique, anti-inflammatoire, anti-resténose et / ou anti-thrombotique est sélectionné parmi le groupe comprenant :
l'abciximab, l'acémétacine, l'acétylvismione B, l'aclarubicine, l'adémétionine, l'adriamycine, l'aescine, l'afromosone, l'akagérine, l'aldesleukine, l'amidorone, l'aminoglutéthimide, l'amsacrine, l'anakinra, l'anastrozole, l'anémonine, l'anoptérine, les antimycotiques, les antithrombotiques, l'apocymarine, l'argatroban, l'aristolactam-All, l'acide aristolochique, l'ascomycine, l'asparaginase, l'aspirine, l'atorvastatine, l'auranofine, l'azathioprine, l'azithromycine, la baccatine, la bafilomycine, le basiliximab, la bendamustine, la benzocaïne, la berbérine, la bétuline, l'acide bétulinique, le bilobol, la bisparthénolidine, la bléomycine, la combrestatine, les acides boswelliques, le brucéanol A, B et C, la bryophylline A, le busulfan, l'antithrombine, la bivalirudine, les cadhérines, la camptothécine, la capécitabine, l'acide o-carbamoyl-phénoxyacétique, le carboplatine, la carmustine, le célécoxib, la cépharanthine, la cérivastatine, les inhibiteurs du CETP, le chlorambucil, le phosphate de chloroquine, la cicutoxine, la ciprofloxacine, le cisplatine, la cladribine, la clarithromycine, la colchicine, la concanamycine, la coumadine, le peptide natriurétique de type C (CNP), la cudraisoflavone A, la curcumine, le cyclophosphamide, la cyclosporine A, la cytarabine, la dacarbazine, le daclizumab, la dactinomycine, la dapsone, la daunorubicine, le diclofénac, la 1,11-diméthoxycanthin-6-one, le docétaxel, la doxorubicine, la daunamycine, l'épirubicine, l'érythromycine, l'estramustine, l'étoposide, le filgrastim, la fluroblastine, la fluvastatine, la fludarabine, le dihydrogénophosphate de fludarabine-5', le fluorouracile, la folimycine, le fosfestrol, la gemcitabine, le ghalakinoside, le ginkgol, l'acide ginkgolique, le glycoside 1a, le 4-hydroxyoxycyclophosphamide, l'idarubicine, l'ifosfamide, la josamycine, le lapachol, la lomustine, la lovastatine, le melphalan, la midécamycine, la mitoxantrone, la nimustine, la pitavastatine, la pravastatine, la procarbazine, la mitomycine, le méthotrexate, la mercaptopurine, la thioguanine, l'oxaliplatine, l'irinotécan, le topotécan, l'hydroxycarbamide, la miltéfosine, la pentostatine, la pégaspargase, l'exémestane, le létrozole, le formestane, le mycophénolate mofétil, la β-lapachone, la podophyllotoxine, le 2-éthylhydrazide de l'acide podophyllique, le molgramostime (rhuGM-CSF), le peginterféron α-2b, le lénograstime (r-HuG-CSF), le macrogol, la sélectine (antagoniste de la cytokine), les inhibiteurs de la cytokinine, l'inhibiteur de COX-2, l'angiopeptine, les anticorps monoclonaux inhibiteurs de la prolifération des cellules musculaires, les antagonistes du bFGF, le probucol, les prostaglandines, la 1-hydroxy-11-méthoxycanthin-6-one, la scopolétine, les donneurs de NO, le tétranitrate de pentaérythrityle et les sydnoimines, le tamoxifène, la staurosporine, le β-estradiol, l'α-estradiol, l'estriol, l'estrone, l'éthynylestradiol, la médroxyprogestérone, les cypionates d'estradiol, les benzoates d'estradiol, le tranilast, la kamébakaurine et d'autres terpénoïdes qui sont utilisés dans le traitement du cancer, le vérapamil, les inhibiteurs de tyrosine-kinase (tyrphostines), le paclitaxel, le 6-α-hydroxy-paclitaxel, les taxotères, la mofébutazone, le lonazolac, la lidocaïne, le kétoprofène, l'acide méfénamique, le piroxicam, le méloxicam, la pénicillamine, l'hydroxychloroquine, l'aurothiomalate de sodium, l'oxacéprol, le β-sitostérol, la myrtécaïne, le polidocanol, le nonivamide, le lévomenthol, l'ellipticine, le D-24851 (Calbiochem), le colcémide, la cytochalasine A-E, l'indanocine, le nocodazole, la bacitracine, les antagonistes du récepteur de la vitronectine, l'azélastine, le stimulateur de guanidylcyclase, l'inhibiteur tissulaire de la métalloprotéinase-1 et -2, les acides nucléiques libres, les acides nucléiques incorporés dans des transmetteurs viraux, les fragments d'ADN et d'ARN, l'inhibiteur-1 de l'activateur du plasminogène, l'inhibiteur-2 de l'activateur du plasminogène, les oligonucléotides antisens, les inhibiteurs du VEGF, l'IGF-1, les principes actifs du groupe des antibiotiques, le céfadroxil, la céfazoline, le céfaclor, la céfoxitine, la tobramycine, la gentamycine, les pénicillines, la dicloxacilline, l'oxacilline, les sulfonamides, le métronidazole, l'énoxaparine, l'héparine, l'hirudine, le PPACK, la protamine, la pro-urokinase, la streptokinase, la warfarine, l'urokinase, les vasodilatateurs, le dipyramidole, le trapidil, les nitroprussiates, les antagonistes du PDGF, la triazolopyrimidine, la séramine, les inhibiteurs de l'ECA, le captopril, le cilazapril, le lisinopril, l'énalapril, le losartan, les inhibiteurs de la thioprotéase, la prostacycline, le vapiprost, l'interféron a, β et γ, les antagonistes de l'histamine, les bloquants de la sérotonine, les inhibiteurs de l'apoptose, les régulateurs de l'apoptose, l'halofuginone, la nifédipine, le tocophérol, le tranilast, la molsidomine, les polyphénols du thé, le gallate d'épicatéchine, le gallate d'épigallocatéchine, le léflunomide, l'étanercept, la sulfasalazine, la tétracycline, la triamcinolone, la mutamycine, le procaïnimide, l'acide rétinoïque, la quinidine, le disopyrimide, le flécaïnide, la propafénone, le sotalol, les stéroïdes naturels et obtenus par synthèse, tels que la bryophylline A, l'inotodiol, le maquiroside A, le ghalakinoside, la mansonine, le strébloside, l'hydrocortisone, la betaméthasone, la dexaméthasone, les substances non stéroïdiennes (AINS), le fénoprofène, l'ibuprofène, l'indométhacine, le naproxène, la phénylbutazone, les agents antiviraux, l'acyclovir, le ganciclovir, la zidovudine, le clotrimazole, la flucytosine, la griséofulvine, le kétoconazole, le miconazole, la nystatine, la terbinafine, les agents antiprotozoaires, la chloroquine, la méfloquine, la quinine, les terpénoïdes naturels, l'hippocaesculine, l'angélate de barringtogénol-C21, la 14-déhydroagrostistachine, l'agroskérine, l'agrostistachine, la 17-hydroxyagrostistachine, les ovatodiolides, l'acide 4,7-oxycycloanisomélique, les baccharinoïdes B1, B2, B3 et B7, le tubéimoside, le brucéantinoside C, les yadanziosides N et P, l'isodéoxyéléphantopine, la tomenphantopine A et B, la coronarine A, B, C et D, l'acide ursolique, l'acide hyptatique A, l'iso-iridogermanal, le maytenfoliol, l'effusantine A, l'excisanine A et B, la longikaurine B, la sculponéatine C, la kamébaunine, la leukaménine A et B, la 13,18-déhydro-6-alpha-sénécioyloxychaparrine, la taxamairine A et B, le régénilol, le triptolide, la cymarine, l'hydroxyanoptérine, la protoanémonine, le chlorure de chéliburine, la sinococuline A et B, la dihydronitidine, le chlorure de nitidine, la 12-β-hydroxypregnadièn-3,20-dione, l'hélénaline, l'indicine, le N-oxyde d'indicine, la lasiocarpine, l'inotodiol, la podophyllotoxine, la justicidine A et B, la larréatine, la mallotérine, le mallotochromanol, l'isobutyrylmallotochromanol, la marchantine A, la maytansine, la lycoridicine, la margétine, la pancratistatine, la liriodénine, l'oxoushinsunine, le périplocoside A, la déoxypsorospermine, la psychorubine, la ricine A, la sanguinarine, l'acide de froment de manwu, la méthylsorbifoline, les chromones de sphathélia, la stizophylline, la dihydrousambaraensine, l'hydroxyusambarine, la strychnopentamine, la strychnophylline, l'usambarine, l'usambarensine, la liriodénine, la daphnorétine, le laricirésinol, le méthoxylaricirésinol, le syringarésinol, le sirolimus (la rapamycine), le biolimus A9, le pimécrolimus, l'évérolimus, le myolimus, le novolimus, le ridaforolimus, le temsirolimus, le zotarolimus, le tacrolimus, le fasudil, les épothilones, la somatostatine, la roxithromycine, la troléandomycine, la simvastatine, la rosuvastatine, la vinblastine, la vincristine, la vindésine, le téniposide, la vinorelbine, le trofosfamide, le tréosulfan, le témozolomide, le thiotépa, la trétinoïne, la spiramycine, l'umbelliférone, la désacétylvismione A, la vismione A et B, la zéorine.

11. Ballonnet de cathéter pour valvuloplastie selon la revendication 10, dans lequel l'agent actif est sélectionné parmi le groupe comprenant :
le paclitaxel, les taxanes, le docétaxel, le sirolimus, le biolimus A9, le pimécrolimus, l'évérolimus, le myolimus, le novolimus, le ridaforolimus, le temsirolimus, le zotarolimus, le tacrolimus, le fasudil et les épothilones.

12. Ballonnet de cathéter pour valvuloplastie selon l'une des revendications de 1 à 11, dans lequel le revêtement comprend en outre de la gomme-laque.

13. Ballonnet de cathéter pour valvuloplastie selon l'une quelconque des revendications de 1 à 12, dans lequel le revêtement comprend en outre une couche de base déposée sur ledit ballonnet de cathéter constituée d'un copolymère greffé d'alcool polyvinylique - polyéthylène glycol et / ou de la gomme-laque.

14. Cathéter à ballonnet comprenant un ballonnet de cathéter pour valvuloplastie selon l'une quelconque des revendications de 1 à 13.

15. Cathéter à ballonnet selon la revendication 14 conçu pour prévenir ou pour réduire la resténose à une valve cardiaque.
